# FASCICULE DE BREVET EUROPEEN

(11) **EP 4 355 271 B1**
(45) Date de publication et mention de la délivrance du brevet: **15.07.2026**
(21) Numéro de dépôt: 22744246.4
(22) Date de dépôt: 16.06.2022
(51) Int. Cl.: A61F 2/30, A61K 35/32, C12N 5/00, C12N 5/077, A61F 2/46, A61L 27/38

(54) **DISPOSITIF IMPLANTABLE COMPRENANT DES MOYENS DE MOBILISATION EXTERNE POUR LA FORMATION D'UN CARTILAGE ARTICULAIRE**
IMPLANTIERBARE VORRICHTUNG MIT EXTERNEN MOBILISIERUNGSMITTELN ZUR GELENKKNORPELBILDUNG
IMPLANTABLE DEVICE COMPRISING EXTERNAL MOBILIZATION MEANS FOR THE FORMATION OF ARTICULAR CARTILAGE

(30) Priorité: 17.06.2021 FR 2106453
(43) Date de publication de la demande: 24.04.2024
(73) Titulaire: Palingen, 34680 Saint-Georges-D'Orques (FR)
(72) Inventeur: POURQUIER, Didier, 34680 Saint-Georges-D'Orques (FR); MOUKOKO, Didier, 49170 Saint-Martin-Du-Fouilloux (FR)
(74) Mandataire: Regimbeau
(86) Numéro de dépôt international: PCT/FR2022/051169
(87) Numéro de publication internationale: WO 2022/263778

(56) Documents cités:
- US-A1- 2001 014 473
- US-A1- 2005 125 077
- US-A1- 2014 335 612
- US-B1- 6 530 956

## Description

### DOMAINE DE L'INVENTION

La présente invention se rapporte à un dispositif implantable prévu pour susciter la formation d'un cartilage articulaire, de préférence adapté à la morphologie d'un cartilage articulaire détérioré d'un individu, afin de remplacer ledit secteur de cartilage détérioré.

L'invention concerne en particulier un dispositif implantable adapté pour favoriser la formation des couches cellulaires spécifiques du cartilage articulaire. Le dispositif implantable est associé à une matrice cellulaire de réparation. La matrice de réparation comprend de préférence un greffon de périoste.

### ETAT DE LA TECHNIQUE

Une chondropathie se manifeste par une désagrégation d'une ou plusieurs surfaces cartilagineuses, qui perdent leurs propriétés mécaniques de protection des surfaces osseuses et d'amortissement des sollicitations mécaniques d'une articulation. Une chondropathie non traitée chez l'humain peut dégénérer en arthrose évoluée, causant d'importantes douleurs chroniques et pouvant être très invalidante notamment pour le patient âgé.

Dans l'exemple du genou, au cours de mouvements naturels de marche ou d'accroupissement, la pression exercée sur l'articulation du genou entre le fémur et le tibia est très importante. Si la fonction de mobilité et d'amortissement du cartilage articulaire n'est pas maintenue, ces mouvements naturels deviennent douloureux.

De telles lésions du cartilage articulaire peuvent se situer sur une seule des deux surfaces articulaires qui se font face, mais les deux surfaces articulaires cartilagineuses en vis-à-vis peuvent aussi être affectées. Les lésions articulaires trouvent leur source soit dans l'usure du cartilage liée à l'âge (arthrose), soit dans des traumatismes articulaires liés à des chocs ou accidents, notamment chez des sujets jeunes. Dans la pratique médicale actuelle, le degré de gravité des chondropathies est évalué sur le plan clinique par le score fonctionnel de l'ICRS, et sur le plan radiologique (IRM) par le grade ICRS ou par la classification d'Outerbridge.

L'état de la technique connu comprend de nombreuses approches thérapeutiques pour les patients atteints de chondropathie. Dans des formes débutantes d'arthrose, des soins sont proposés à base d'anti-inflammatoires par voie générale ou par injection locale. On connaît également des injections intra-articulaires, par exemple des injections d'acide hyaluronique (on parle alors de « visco-supplémentation ») ou encore de plasma enrichi en plaquettes. Des approches thérapeutiques relativement peu invasives sous arthroscopie peuvent aussi être proposées, comme le lavage articulaire avec débridement, la technique de microperforations de Pridie, la technique de micro-fractures, ou encore l'utilisation de matrices tridimensionnelles composées de collagène et d'hydroxyapatite.

Cependant, les approches susmentionnées fournissent des résultats souvent transitoires au niveau clinique, et peu probants pour la formation de nouveau cartilage. Sur le plan histologique, le cartilage ainsi formé est de mauvaise qualité.

Une autre approche connue consiste à implanter directement un ou plusieurs secteurs ostéo-cartilagineux sains, issus d'une articulation d'un individu à traiter, dans le secteur d'articulation à traiter chez le même individu (par « autogreffe ostéochondrale »). Dans ce cas, des carottes ostéo-cartilagineuses sont prélevées par exemple sur des zones non porteuses de l'articulation à traiter, et sont transférées vers le secteur nécessitant une réparation.

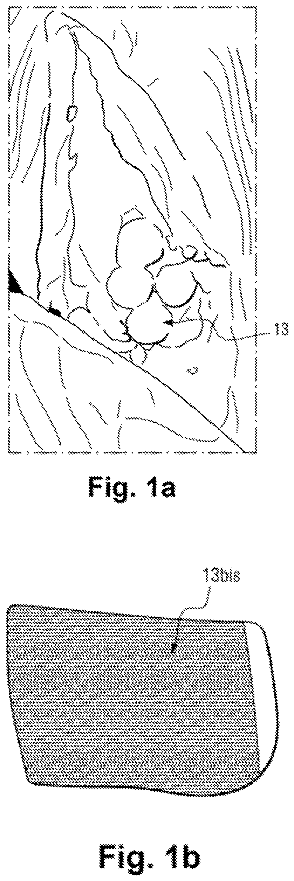

On a représenté sur la Figure 1a une autogreffe ostéochondrale au niveau d'un condyle fémoral, pour traiter l'articulation du genou d'un individu humain *(Reprise du sport après autogreffe ostéochondrale en mosaïque des condyles fémoraux :* 25 *cas* à 9 *ans de recul moyen,* Cognault, Seurat, Chaussard, lonescu, Saragaglia, Revue de chirurgie orthopédique et traumatologique 101 (2015) pp. 215-220).

La Figure 1a illustre une technique de « mosaïcplastie ». Une pluralité de carottes 13 (ici quatre carottes) préalablement prélevées chez le même individu sont implantées, selon une disposition en mosaïque, dans la surface articulaire endommagée. Chaque carotte 13 comprend une surface cartilagineuse et une épaisseur sous-jacente d'os sous-chondral. De préférence, les carottes 13 présentent un diamètre d'environ 7 ou 8 millimètres et une longueur d'environ 15 millimètres.

Cependant, le nombre de sites « donneurs », c'est-à-dire de zones non porteuses d'articulation appropriées pour le prélèvement des carottes, est limité. Cette technique d'autogreffe par mosaïcplastie s'adresse donc au traitement de lésions articulaires de surface limitée (typiquement inférieure à 3 centimètres carrés).

Selon une autre approche dite « allogreffe ostéochondrale », un secteur ostéo-cartilagineux sain issu d'un individu différent de l'individu à traiter. Cette technique est appropriée pour traiter des lésions articulaires de surface plus importante, en utilisant des greffons ostéo-cartilagineux plus volumineux.

Cependant, cette technique est difficile à mettre en œuvre en pratique courante, car le prélèvement ostéo-cartilagineux doit être réalisé sur un organisme humain décédé moins de 12 heures après le décès.

La Figure 1b représente un exemple de carotte ostéo-cartilagineuse 13bis, destinée par exemple à une allogreffe ostéochondrale articulaire. La carotte 13bis est ici représentée isolée, après prélèvement et avant implantation chez le patient. La longueur de la carotte 13bis est ici d'environ 4 centimètres.

Compte-tenu des limites des approches présentées ci-avant en relation aux Figures 1a et 1b, il a été proposé de procéder à une arthroplastie (pose d'une prothèse articulaire) en cas d'arthrose évoluée chez l'humain. Les articulations fréquemment traitées de cette manière comprennent notamment les articulations de la hanche et du genou. Pour une chondropathie évoluée du genou, il est fréquent d'implanter chirurgicalement une prothèse définitive unicompartimentale ou totale de genou, fixée à demeure sous anesthésie.

Le document US 2001/0014473 décrit un dispositif de production in vitro de tissue cartilagineux qui définit un espace cellulaire et qui est destiné à être introduit dans un environnement de culture.

La demande internationale de brevet publiée sous le numéro WO 2005/016175 A2 décrit une prothèse articulaire définitive conçue dans un matériau biocompatible rigide, implantée directement dans la cavité articulaire affectée, pour restaurer les propriétés mécaniques attendues de l'articulation. Cependant, dans le cas d'une telle prothèse articulaire permanente, une longue rééducation de l'articulation doit être mise en œuvre. Une prise en charge médicale importante dans un centre spécialisé, comprenant des séances de kinésithérapie et des soins de support, est souvent nécessaire.

Il existe en outre des risques importants de perte de fonctionnalité mécanique de la prothèse articulaire au cours du temps, par usure mécanique des composants de la prothèse, et/ou par infection du matériel prothétique.

Il résulte de ce qui précède qu'en l'état, les pratiques chirurgicales et approches actuelles ne permettent pas une réparation entièrement satisfaisante, suffisamment qualitative et durable, du cartilage articulaire, et/ou présentent des risques d'infection et/ou d'usure mécanique des dispositifs implantés. En outre, certains de ces traitements de l'état de la technique connu nécessitent une lourde prise en charge médico-chirurgicale.

Les approches connues ne répondent donc pas de manière appropriée aux enjeux de santé publique, concernant le soin des douleurs articulaires et des troubles de mobilité articulaire liés aux lésions du cartilage.

### DESCRIPTION GENERALE DE L'INVENTION

Il existe donc un besoin pour un dispositif implantable capable de promouvoir la génération d'un nouveau secteur de cartilage articulaire, afin de remplacer une zone de cartilage détériorée d'une articulation, en particulier au niveau du genou (articulation fémoro-tibiale, ou fémoro-patellaire) ou au niveau de l'articulation de la hanche chez l'être humain.

Le dispositif recherché doit permettre la formation d'un nouveau tissu ostéo-cartilagineux, comprenant avantageusement un secteur de cartilage articulaire et une couche d'os sous-chondral.

Pour obtenir un tissu ostéo-cartilagineux de bonne qualité, le cartilage néoformé doit comporter les différentes couches de chondrocytes (couches superficielle, transitionnelle, radiaire, cartilage calcifié) caractéristiques du cartilage articulaire. Ces différentes couches fonctionnelles du cartilage articulaire sont décrites, en termes de structure et de composition cellulaire, dans la publication suivante : Composition and Structure of Articular Cartilage: A Template for Tissue Repair, Poole, Kojima, Yasuda, Mwale, Kobayashi, Laverty, Octobre 2001, Clinical Orthopaedics and Related Research 391(391):S26-S33.

Le dispositif recherché doit présenter une excellente manœuvrabilité chirurgicale, et ne pas nécessiter une intervention médicale trop lourde pour son implantation. De préférence, le dispositif doit permettre de réparer des zones articulaires de surface plus vaste en comparaison aux techniques d'autogreffe ostéochondrale, tout en étant facile à implanter.

De manière préférée, le dispositif recherché doit susciter la formation d'un nouveau secteur de cartilage dont la géométrie soit bien adaptée à la morphologie spécifique de l'articulation à traiter. On souhaite que la surface du cartilage réparé présente une géométrie très proche de la géométrie du cartilage natif, ce qui permet de remplir des critères géométriques de fonctionnement optimal et d'éviter une nouvelle usure du cartilage du patient après traitement.

Un objectif secondaire est de fournir un dispositif permettant la production et la pose de portions de cartilage néoformé dans deux zones de cartilage abîmées, sur les deux faces opposées d'une même articulation lésée.

Pour répondre aux besoins susmentionnés, un premier aspect de l'invention concerne un dispositif implantable amovible destiné à la production de cartilage articulaire, comprenant :
une première partie de support en matériau biocompatible,
une deuxième partie de support en matériau biocompatible montée mobile sur la première partie de support, la première partie de support et la deuxième partie de support définissant entre elles une cavité formant un espace de développement cellulaire,
l'espace de développement cellulaire étant prévu pour recevoir des cellules ostéochondrogéniques se multipliant dans l'espace de développement cellulaire,
et des moyens de mobilisation externe actionnables configurés pour déplacer la deuxième partie de support par rapport à la première partie de support, de sorte à générer un cisaillement au sein de l'espace de développement cellulaire.

Le dispositif implantable tel que défini ci-avant est prévu pour permettre la formation d'un tissu ostéo-cartilagineux autologue, après avoir été implanté dans une région anatomique préférentiellement différente de l'articulation à traiter.

Ce dispositif implantable est amovible, et est implanté de manière transitoire dans un espace tissulaire receveur, de préférence une région intra-musculaire ou une région sous-cutanée de l'individu qui est donc préférentiellement séparée de l'articulation à traiter.

Un avantage d'un tel dispositif est de permettre, à l'issue d'une phase de mobilisation externe du dispositif, la formation d'un tissu ostéo-cartilagineux de géométrie adaptée à la surface du cartilage devant être réparé. Le tissu formé peut ensuite être extrait des supports biocompatibles qui ont servi à sa production et implanté dans l'articulation à traiter.

Le tissu ostéo-cartilagineux formé est par exemple implanté dans l'articulation à traiter de façon similaire aux techniques d'autogreffe ou d'allogreffe mentionnées ci-avant.

De manière optionnelle et non limitative, le dispositif implantable selon ce premier aspect peut présenter les caractéristiques suivantes, prises seules ou en l'une quelconque des combinaisons techniquement possibles :
- le dispositif comprend un boîtier fermé, formé par la première partie de support et par la deuxième partie de support, l'espace de développement cellulaire étant défini à l'intérieur du boîtier.
- la première partie de support comprend une face interne faisant face à la deuxième partie de support, la deuxième partie de support étant configurée pour glisser par rapport à la première partie de support le long de la face interne.
- la deuxième partie de support est montée mobile en translation par rapport à la première partie de support, le long d'une direction parallèle à une surface d'extension de la face interne.
- la deuxième partie de support est montée mobile en rotation par rapport à la première partie de support autour d'un axe de rotation.
- au moins l'une parmi la première partie de support et/ou la deuxième partie de support comprend une face latérale de forme cylindrique s'étendant autour de l'axe de rotation.
- la partie de support comprend en outre une base sensiblement perpendiculaire à la face latérale, les moyens de mobilisation externe comprenant une poignée solidaire en rotation avec la base et mobile en rotation autour de l'axe de rotation.
- les moyens de mobilisation externe comprennent au moins un lacet de mobilisation monté sur la deuxième partie de support, configuré pour tracter la deuxième partie de support.
- le lacet de mobilisation est monté sur une première face externe de la deuxième partie de support.
- le dispositif comprend un lien de mobilisation supplémentaire monté sur une deuxième face externe de la deuxième partie de support, la deuxième face externe étant opposée à la première face externe.
- le lacet de mobilisation comprend un brin semi-rigide, de préférence un brin en matériau polymère.
- le dispositif comprend une glissière configurée pour guider un déplacement de la deuxième partie de support par rapport à la première partie de support.
- au moins l'une parmi la première partie de support et/ou la deuxième partie de support comprend une grille.
- la grille définit une pluralité de cavités de croissance cellulaire débouchant d'un côté intérieur du dispositif.
- au moins l'une parmi la première partie de support et/ou la deuxième partie de support comprend un réseau d'éléments de rigidification fixés à ladite partie de support.
- une rigidité contre le cisaillement au niveau des éléments de rigidification est strictement supérieure à une rigidité contre le cisaillement au niveau d'une zone centrale de l'espace de développement cellulaire.

Un deuxième aspect de l'invention concerne un implant pour la formation de cellules de cartilage, l'implant comprenant :
un dispositif implantable tel que défini ci-avant ;
une matrice cellulaire de réparation, intercalée entre la première partie de support du dispositif implantable et la deuxième partie de support du dispositif implantable, la matrice cellulaire de réparation comprenant un volume de cellules ostéochondrogéniques prévu pour se multiplier dans l'espace de développement cellulaire du dispositif implantable.

De manière optionnelle et non limitative, l'implant selon ce deuxième aspect peut présenter les caractéristiques suivantes, prises seules ou en l'une quelconque des combinaisons techniquement possibles :
- la matrice cellulaire de réparation comprend au moins un greffon de périoste.
- le greffon de périoste est vascularisé.
- le greffon de périoste est issu d'un périoste de tibia.
- l'implant est configuré pour être implanté de manière amovible dans une région intra-musculaire d'un individu.
- l'implant est configuré pour être implanté de manière amovible dans une région sous-cutanée d'un individu.

### DESCRIPTION GENERALE DES FIGURES

D'autres caractéristiques, buts et avantages de l'implant ressortiront de la description qui suit, qui est purement illustrative et non limitative et doit être lue en regard des dessins annexés parmi lesquels, en complément de la Figure 1a et de la Figure 1b déjà commentées :

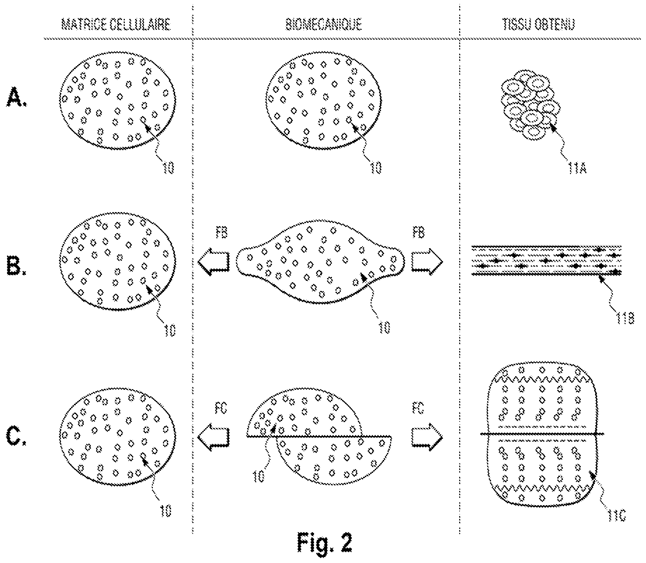

La Figure 2 illustre de manière schématique trois voies de différenciation des cellules souches mésenchymateuses : vers du tissu osseux ou fibreux ou cartilagineux articulaire, en fonction des contraintes mécaniques exercées sur les cellules souches mésenchymateuses.

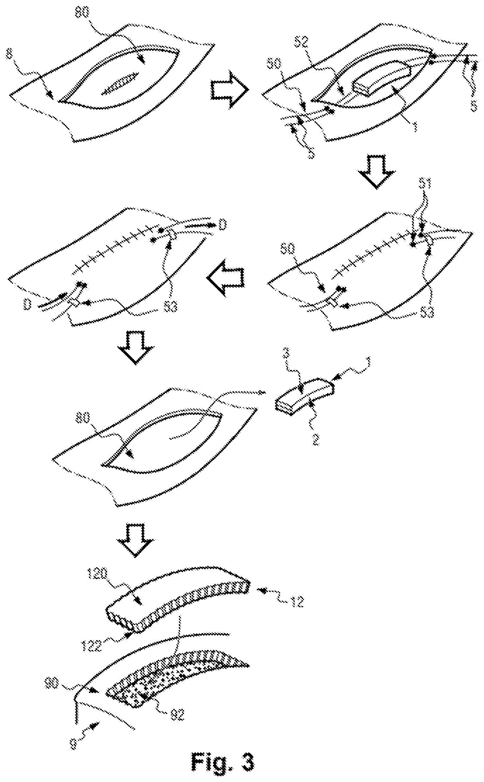

La Figure 3 illustre schématiquement plusieurs étapes successives d'implantation d'un implant selon un exemple de l'invention, dans une région intra-musculaire, puis de mobilisation par des liens externes dudit implant afin de promouvoir la formation d'un nouveau cartilage, puis l'explantation du dispositif et le transfert de la structure ostéo-cartilagineuse produite vers l'articulation receveuse.

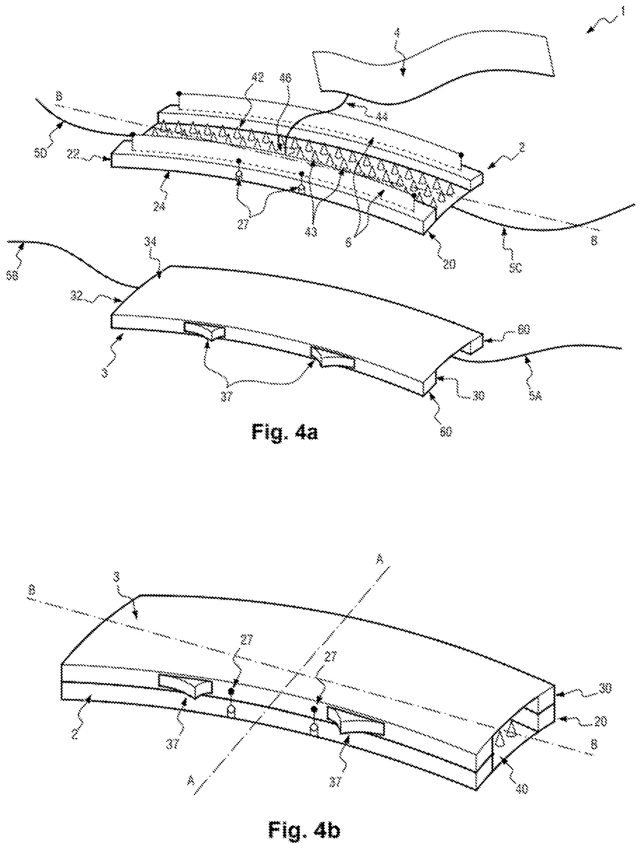

La Figure 4a est une vue schématique de dessus en perspective d'un dispositif implantable selon un premier exemple de réalisation, le dispositif présentant une forme générale de boîtier parallélépipédique, une première portion du dispositif et une deuxième portion du dispositif étant représentées séparément l'une de l'autre. Un greffon de périoste pédiculé est ici monté sur la première portion. La Figure 4b illustre le même dispositif implantable vu en position fermée, la première portion et la deuxième portion étant emboîtées l'une sur l'autre.

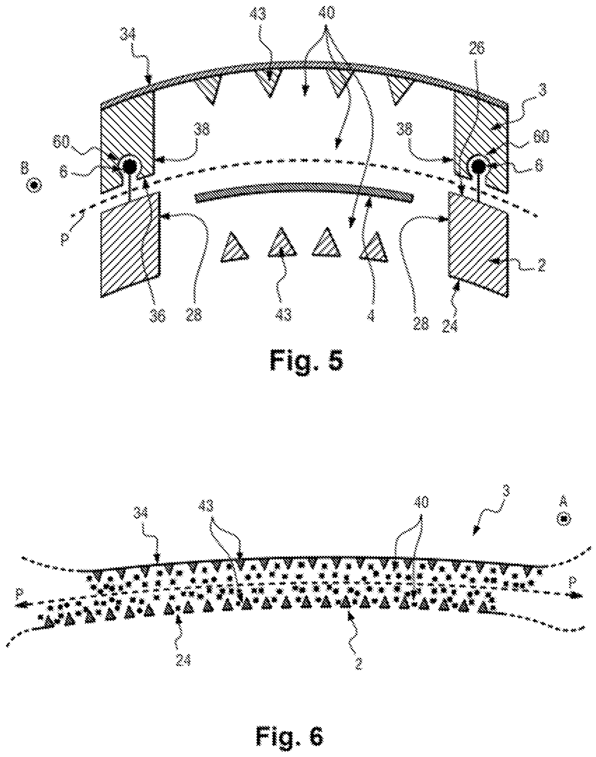

La Figure 5 est une vue schématique en coupe transversale (selon le plan A-A représenté sur la Figure 4b) du dispositif de la Figure 4b, alors qu'un greffon de périoste est disposé dans l'espace de développement cellulaire.
La Figure 6 est une vue schématique en coupe longitudinale (selon le plan B-B représenté sur la Figure 4a) du dispositif de la Figure 4b, illustrant un mouvement relatif de la première portion de support et de la deuxième portion de support ainsi que le développement de cellules ostéochondrogéniques.

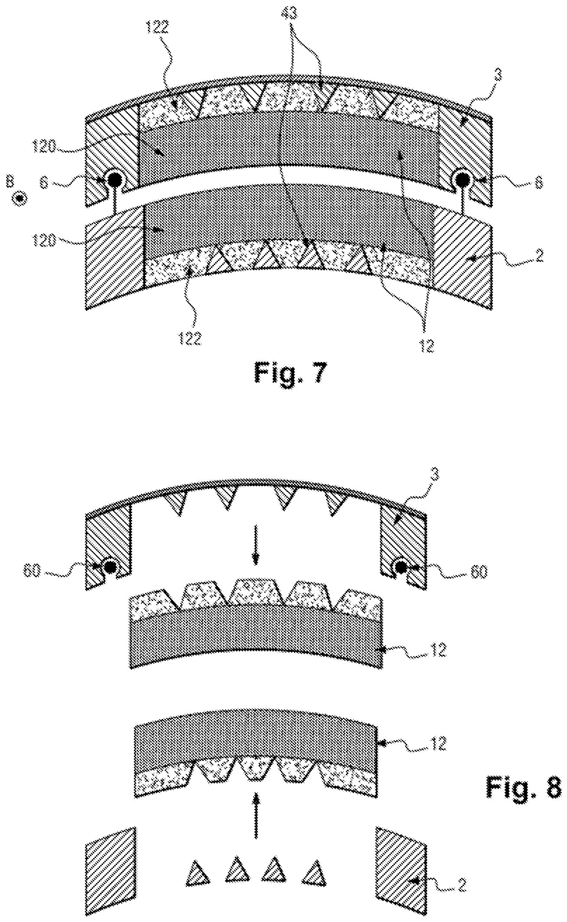

La Figure 7 est une vue schématique en coupe transversale du dispositif de la Figure 4b après mobilisation mécanique dudit dispositif implantable, avec une production de deux portions de tissu ostéo-cartilagineux à l'intérieur du dispositif l'une étant de forme convexe, l'autre étant de forme concave.
La Figure 8 illustre de manière schématique la dissociation d'une première partie de support, d'une deuxième partie de support et de deux portions de tissu ostéo-cartilagineux néoformées qui sont extraites des deux parties de support, le dispositif implantable étant conforme à l'exemple des Figures 4a et 4b.

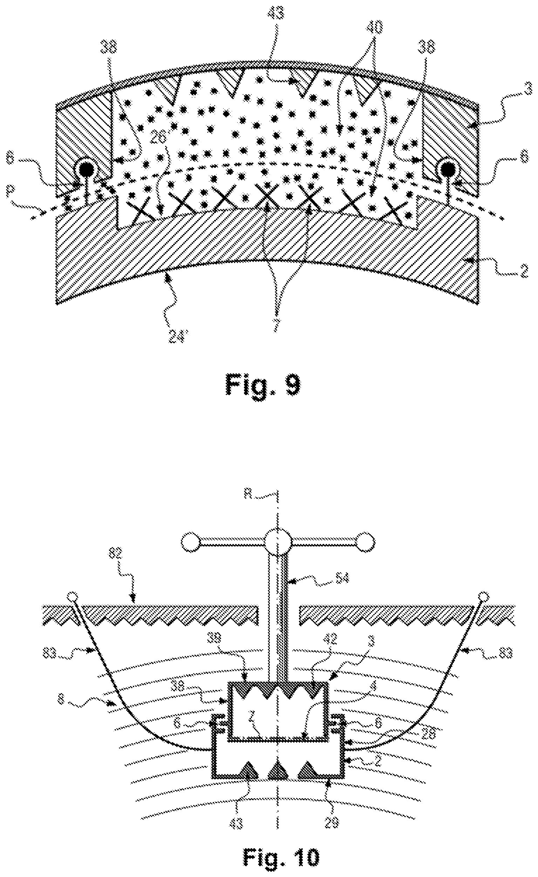

La Figure 9 est une vue schématique en coupe transversale d'un dispositif implantable selon un deuxième exemple de réalisation, le dispositif présentant une forme de boîtier parallélépipédique avec des courbures, et étant destiné à produire une unique portion de tissu ostéo-cartilagineux de surface concave au niveau de l'élément supérieur.
La Figure 10 est une vue schématique en coupe longitudinale d'un dispositif implantable selon un troisième exemple de réalisation, au moment de son implantation, le dispositif présentant une forme générale de boîtier cylindrique et étant implanté dans un muscle.
Ledit dispositif est destiné à produire deux portions de tissu ostéo-cartilagineux néoformées ayant une forme plate, en vis-à-vis.

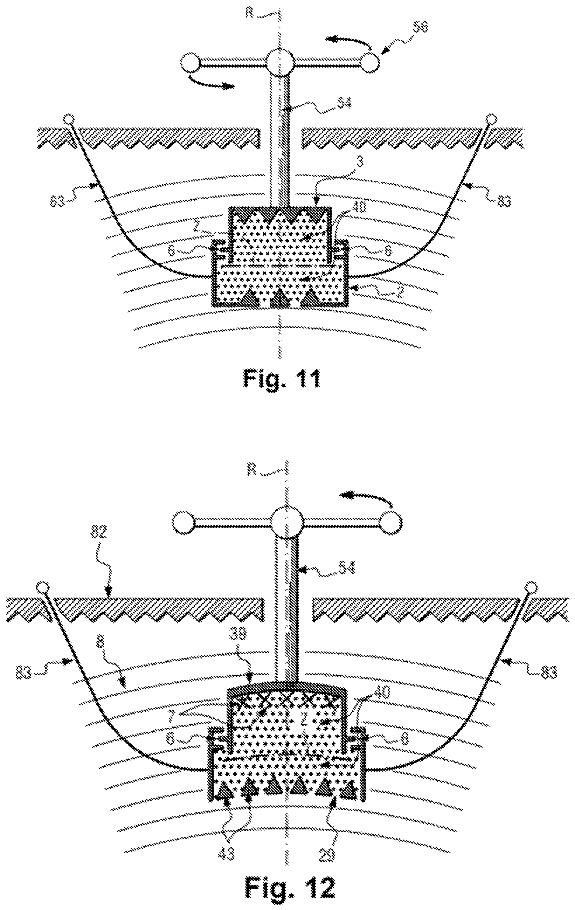

La Figure 11 est une vue schématique en coupe longitudinale du dispositif de la Figure 10 au cours de sa mobilisation mécanique, alors que les cellules ostéochondrogéniques se sont développées.
La Figure 12 est une vue schématique en coupe longitudinale d'un dispositif implantable selon un quatrième exemple de réalisation, le dispositif présentant une forme générale de boîtier quasi-cylindrique dont une base est convexe et l'autre base est concave. Ledit dispositif est destiné à produire une unique portion de tissu ostéo-cartilagineux néoformée de surface convexe au niveau de l'élément inférieur.

### DESCRIPTION DETAILLEE DE PLUSIEURS MODES DE REALISATION DE L'INVENTION

Les exemples de dispositifs implantables décrits ci-après sont préférentiellement prévus pour la formation d'un nouveau cartilage qui sera implanté ultérieurement au niveau du genou (articulation fémoro-tibiale, ou fémoro-patellaire), ou au niveau de l'articulation de la hanche, notamment chez l'être humain.

Les différents exemples de dispositifs implantables décrits ci-après sont cependant adaptables pour promouvoir la génération d'un nouveau secteur de cartilage pour toute surface articulaire du corps humain ou animal.

Les exemples ci-après prévoient l'utilisation d'un greffon de périoste comme matrice de réparation propre à engendrer des cellules ostéochondrogéniques, à l'intérieur d'un espace ménagé dans le dispositif implantable. Comme il sera vu ci-après, le greffon de périoste utilisé est préférentiellement prélevé chez le même individu, et est vascularisé ou non.

On notera toutefois que d'autres types de matrices cellulaires de réparation connus en ingénierie tissulaire peuvent être utilisés. La matrice de réparation peut être associée ou non à des substances ou compositions aidant à la croissance et à la différenciation dirigée vers des cellules de cartilage, telles que des protéines, des facteurs de croissance, etc.

Sur l'ensemble des figures annexées et tout au long de la description ci-après, les éléments similaires portent des références alphanumériques identiques.

### Principes généraux relatifs à la formation d'un nouveau cartilage articulaire

Le cartilage articulaire est un tissu conjonctif souple présent au niveau des articulations, assurant des fonctionnalités mécaniques de protection des extrémités osseuses, d'amortissement de la pression des charges exercées sur les extrémités osseuses et de glissement harmonieux des surfaces articulaires en vis-à-vis.

Le cartilage articulaire est principalement constitué de cellules appelées chondrocytes, et d'une matrice extracellulaire (collagènes, glycosaminoglycanes). Les chondrocytes ont une fonction de synthèse et de maintien du tissu cartilagineux. Ils se situent dans des logettes qui contiennent chacune un ou plusieurs chondrocytes. Ces logettes sont appelées chondroplastes. Des secteurs de cartilage peuvent être détériorés par diverses pathologies touchant le cartilage. La réparation naturelle du cartilage articulaire est très peu efficace. L'évolution des lésions cartilagineuses associe des fissures du cartilage, une diminution de son épaisseur, et des lésions de l'os sous-chondral. Cette évolution peut aboutir à un effacement complet du cartilage avec mise à nu de l'os sous-chondral (éburnation).

Le cartilage articulaire est notamment présent au niveau des surfaces épiphysaires des os longs. Les chondrocytes sont issus de la différenciation cellulaire de cellules souches situées chez l'adulte dans la couche la plus superficielle du cartilage articulaire. Le renouvellement des chondrocytes est cependant très lent, ce tissu se réparant très difficilement.

Les os longs, à l'exception des zones de cartilage, sont recouverts en surface d'une membrane appelée « périoste » assurant la réparation osseuse en cas de fracture.

Le périoste est particulièrement riche en cellules souches réparatrices des tissus squelettiques (cellules souches mésenchymateuses ostéochondrogéniques). Pour une description détaillée de la composition et du rôle du périoste, on pourra se référer à la publication suivante : Periosteum contains skeletal stem cells with high bone regenerative potential controlled by Periostin, Duchamp de Lageneste et al., Nature Communications, février 2018 22;9(1):773.

Face à la difficulté pour l'organisme de réparer spontanément le cartilage articulaire au niveau de zones abîmées de la surface articulaire, le dispositif proposé ici vise à reconstituer dans la zone détériorée un tissu ostéo-cartilagineux qualitativement et géométriquement équivalent au cartilage articulaire natif (tel qu'il était initialement avant détérioration). La reconstitution du tissu ostéo-cartilagineux est réalisée à partir de cellules ostéochondrogéniques (typiquement issues d'un greffon de périoste autologue, comme il sera vu ci-après).

La différenciation des cellules souches mésenchymateuses vers les différents types de tissus conjonctif dépend notamment des contraintes mécaniques dans l'environnement des cellules souches mésenchymateuses. L'étude de ces contraintes rentre dans le cadre plus général de la « mécanobiologie » étudiant l'influence des facteurs mécaniques locaux sur les voies de différenciation cellulaires.

A titre illustratif, on a schématisé sur la Figure 2 annexée trois voies A, B et C de différenciation de cellules souches mésenchymateuses.

A partir d'un volume de cellules ostéochondrogéniques encore immatures 10, il est possible d'influer sur la voie de différenciation et donc sur le phénotype et la fonctionnalité future du tissu néoformé, par le biais des contraintes mécaniques exercées dans le volume cellulaire.

Sur la « voie A », les cellules sont « libres » et aucune contrainte mécanique (ou des contraintes minimes) sont exercées. Les cellules ostéochondrogéniques engendrent alors préférentiellement des cellules osseuses 11A (ostéocytes). Une illustration clinique de cette voie A est le devenir du foyer de fracture osseuse correctement stabilisé par un plâtre ou par un montage orthopédique. Le foyer de fracture (cal de fracture), au sein duquel des cellules ostéochondrogéniques issues du périoste ou de la moelle osseuse sont présentes précocement, va se différencier en os (en général après un passage par l'état de cartilage endochondral).

Sur la « voie B », des forces FB d'étirement sont exercées selon une direction privilégiée. On observe alors la formation d'un tissu fibreux 11B (tissu constitutif des ligaments). Une illustration clinique de cette voie B est par exemple la reconstruction chirurgicale du ligament latéral externe de la cheville par lambeau de périoste prélevé sur le péroné.

Enfin, sur la « voie C », des forces FC de cisaillement sont exercées : la partie haute du volume des cellules ostéochondrogéniques est déplacée à gauche, et la partie basse dudit volume est déplacée vers la droite. A la connaissance de la Demanderesse, cette voie C de différenciation favorisant l'ostéochondrogénèse n'a pas été isolée à ce jour, de façon naturelle ou en pratique médico-chirurgicale. Le dispositif proposé s'appuie ainsi sur des mouvements de cisaillement appliqués au volume cellulaire, favorisant une différenciation dirigée des cellules ostéochondrogéniques vers la formation de chondrocytes, pour obtenir à terme des secteurs de cartilage articulaire.

Après différenciation dirigée des cellules ostéochondrogéniques par la voie C, on obtient ici de nouvelles surfaces cartilagineuses 11C s'étendant de part et d'autre le long du plan de clivage.

Les différents exemples de dispositif implantable décrits ci-après visent à pousser la différenciation dirigée des cellules ostéochondrogéniques immatures, dans une voie de différenciation cellulaire similaire à la voie C susmentionnée.

Comme il sera vu ci-après, le dispositif implantable de l'invention définit un espace de développement cellulaire dans lequel des cellules ostéochondrogéniques immatures sont amenées à se multiplier. Une matrice de réparation, comprenant typiquement un greffon de périoste (de préférence autologue) est disposée dans cet espace. Des contraintes mécaniques sont générées, par mobilisation externe, à l'intérieur de cet espace ; engendrant un cisaillement de la matrice cellulaire de réparation selon un plan de clivage précis, reproductible à l'identique à chaque mouvement.

Afin d'assurer le développement des cellules ostéochondrogéniques et la différenciation dirigée desdites cellules vers des chondrocytes (voie C), il est ici proposé de poser le dispositif implantable, de manière temporaire directement dans l'organisme de l'individu à traiter, dans un espace tissulaire favorable à ce développement cellulaire.

L'espace sélectionné (par exemple une région musculaire ou une région sous-cutanée) nourrit la matrice cellulaire de réparation disposée à l'intérieur du dispositif implantable, pour favoriser le développement des cellules ostéochondrogéniques. Il est particulièrement pertinent d'implanter le dispositif dans un environnement capable de nourrir ainsi la matrice cellulaire de réparation, dans le cas où cette dernière comprend un greffon de périoste non vascularisé.

De manière très préférentielle, un dispositif implantable conforme à l'un quelconque des exemples décrits ci-après n'est pas implanté initialement dans l'articulation à traiter ou au voisinage de l'articulation. Il est proposé de poser ledit dispositif implantable, de manière temporaire, dans une autre région de l'organisme de l'individu.

Il est très avantageux de poser ledit dispositif implantable de manière amovible dans une région intra-musculaire de l'individu, ou dans une région sous-cutanée de l'individu, au cours de la phase de croissance et de différenciation dirigée des cellules ostéochondrogéniques.

La région intra-musculaire présente un environnement biologique particulièrement favorable à la croissance de nouvelles cellules ostéochondrogéniques. Concernant les interactions entre l'environnement de la région intra-musculaire et la croissance des cellules ostéochondrogéniques, on peut citer la publication suivante : The potential role of muscle in bone repair, Liu, Schindeler, Little, J Musculoskelet Neuronal Interact 2010 ; 10(1) pp.71-76, ainsi que la publication suivante : Role of muscle stem cells during skeletal regeneration, Rana Abou-Khalil , Frank Yang, Shirley Lieu, Anaïs Julien, Jaselle Perry, Catia Pereira, Frédéric Relaix, Théodore Miclau, Ralph Marcucio, Céline Colnot, Stem Cells, Mai 2015, 33(5):1501-11. Ces deux publications scientifiques montrent que la région intra-musculaire présente non seulement une vascularisation élevée permettant de fournir un afflux de sang nourrissant le greffon, mais contient également des cellules progénitrices contribuant de manière directe à la réparation osseuse.

Il est ainsi avantageux d'utiliser un greffon de périoste comme matrice de réparation à l'intérieur du dispositif implantable, et de placer le dispositif implantable à l'intérieur d'une région intra-musculaire. L'action combinée des cellules de périoste et des cellules myogéniques avoisinantes forme un contexte particulièrement propice à la multiplication et à la différenciation dirigée des cellules ostéochondrogéniques, pour former le tissu ostéo-cartilagineux comprenant le nouveau secteur de cartilage et l'os sous chondral.

On décrit ci-après les principales étapes de la pose de dispositif implantable et de l'obtention d'un nouveau secteur de cartilage, selon un exemple.

*Pose d'un implant et mobilisation mécanique afin de promouvoir la formation de cartilage* On a représenté sur la Figure 3 annexée des étapes successives de pose, ici dans une région 80 intra-musculaire d'un muscle 8 d'un individu humain, d'un implant comprenant un dispositif 1 implantable amovible et comprenant une matrice 4 de réparation cellulaire, puis de mobilisation externe dudit implant. Enfin, on extrait du dispositif 1 le cartilage néoformé 12 et on implante le cartilage néoformé 12 dans un secteur de surface articulaire (ici dans la cavité de parage 92) d'un secteur de cartilage endommagé 90 du même individu.

Le dispositif 1 implantable présente ici une forme de boîtier parallélépipédique. Il est par exemple conforme à l'un quelconque des exemples 1A et 1B décrits ci-après. Les mêmes étapes de procédé pourraient être mises en œuvre à l'aide d'un autre dispositif implantable.

La surface articulaire à traiter est de préférence séparée du muscle 8. Autrement dit, il n'y a pas nécessairement de lien entre la surface articulaire endommagée à traiter et le muscle sélectionné pour la pose du dispositif 1 implantable, excepté que le muscle appartient très avantageusement au même individu que la surface articulaire, afin d'éviter de potentiels phénomènes de rejet du cartilage néoformé après implantation.

Dans le présent exemple, le muscle 8 est un mollet. La région intra-musculaire 80 correspond par exemple au secteur central du mollet.

Pour commencer la pose, la région intra-musculaire 80 est mise à nu. Une incision est par exemple pratiquée dans le muscle 8. Un espace suffisant pour insertion du dispositif 1 est ménagé au sein du muscle par le praticien.

Le dispositif 1 est ensuite inséré dans la région intra-musculaire 80. Le dispositif 1 est associé à une matrice de réparation (non représentée sur la Figure 3) insérée dans un espace de développement cellulaire à l'intérieur du dispositif 1, formant un implant pour la formation de cartilage. Le dispositif 1 est typiquement refermé autour de la matrice de réparation.

Un rôle de la matrice de réparation est de fournir des cellules ostéochondrogéniques prévues, après multiplication et différenciation dirigée, pour se différencier en chondrocytes et former un cartilage néoformé 12. De manière très préférentielle, la matrice de réparation comprend un greffon de périoste.

Le greffon de périoste, de préférence autologue, a par exemple été prélevé sur une surface osseuse de l'individu. Dans le présent exemple, on prélève le greffon de périoste sur la face antérieure du tibia. Par exemple, le greffon de périoste est un lambeau de forme rectangulaire, très peu épais, présentant une largeur par exemple comprise entre 1 centimètre et 5 centimètres et une longueur comprise entre 5 centimètres et 10 centimètres. En alternative, le greffon de périoste peut être issu de tout autre os de l'individu. Il peut être ou non associé à un pédicule 44 (on parlera alors d'un « lambeau » de périoste) ;

Le dispositif 1 est associé à des moyens de mobilisation externe, permettant de forcer le déplacement d'une première portion 2 du dispositif 1 par rapport à une deuxième portion 3 du dispositif 1. Un cisaillement est ainsi généré entre les deux dites portions, pour générer des contraintes de cisaillement au sein de la matrice de réparation cellulaire.

Dans le présent exemple, les moyens de mobilisation externe comportent des liens de mobilisation 5. Ici, une première paire de liens de mobilisation 5 est fixée de chaque côté de la deuxième portion 3, et une deuxième paire de liens est fixée de chaque côté de la première portion 2.

Cette deuxième paire de liens permet de stabiliser sur place la première portion 2 par rapport à la région intra-musculaire, alors que les liens de mobilisation de la première paire de liens sont utilisés pour mobiliser la deuxième portion 3. Pour stabiliser les liens de la deuxième paire par rapport à la région intra-musculaire (notamment au cours de la mobilisation du dispositif 1), de préférence, des attaches 53 fixent lesdits liens contre la peau. Ces attaches 53 sont de préférence réalisées lors de la fermeture de l'incision du muscle 8, par exemple par des sutures.

On peut envisager l'utilisation d'un nombre de paires de liens de stabilisation ou de mobilisation supérieur à deux, si nécessaire.

De retour à la Figure 3, pour permettre la mobilisation ultérieure après suture de la région 80 intra-musculaire, les liens de mobilisation 5 comportent ici une partie interne 52 sous-cutanée et une partie externe 50 destinée à demeurer à l'extérieur de la peau de l'individu. On choisit des liens de mobilisation 5 de longueur suffisante pour disposer de parties externes 50 d'une longueur suffisante pour en assurer une manipulation aisée.

Ici, les liens de mobilisation 5 comprennent de préférence des brins semi-rigides, de préférence des fils plastifiés et/ou des fils de suture.

L'incision du muscle 8 est ensuite refermée de sorte à laisser les parties externes 50 à l'extérieur. Les parties externes 50 passent par des points d'incision 51 pratiqués dans la peau, de préférence au-dessus du muscle 8. On réalise alors les attaches 53 le cas échéant.

Le dispositif 1 est formé dans un ou plusieurs matériaux biocompatibles, et la matrice de réparation est de préférence autologue. Les risques de rejet du dispositif 1 par l'organisme sont donc limités.

A ce stade, le dispositif 1 implantable est de préférence laissé immobile, à demeure dans la région 80 intra-musculaire, dans un contexte d'immobilité du muscle. Un avantage est de laisser du temps aux cellules ostéochondrogéniques pour se multiplier au sein de l'espace de développement cellulaire, à l'intérieur du dispositif 1. Les cellules ostéochondrogéniques se multiplient alors en-dehors de toute contrainte mécanique de cisaillement, jusqu'à « coloniser » l'espace de développement cellulaire.

A une étape suivante, on mobilise (de préférence mécaniquement) le dispositif 1 pour mettre en mouvement la deuxième portion 3 de boîtier par rapport à la première portion 2 de boîtier. Avant mobilisation, les cellules ostéochondrogéniques se trouvent généralement dans un état encore relativement immature, permettant leur différenciation dirigée.

Dans l'exemple illustré sur la Figure 3, durant la phase de mobilisation externe :
- La première portion 2 de boîtier demeure fixe par rapport au muscle 8. On tient par exemple les liens de mobilisation 5 attachés à la première portion 2, pour forcer la première portion 2 à demeurer fixe par rapport au muscle.
- La deuxième portion 3 de boîtier est déplacée par rapport au muscle 8 et par rapport à la portion 2, *via* les liens de mobilisation 5 attachés à la deuxième portion 3. On tire par exemple sur un lien de mobilisation 5 situé sur un côté de la deuxième portion 3, par exemple selon la direction D de mobilisation illustrée sur la Figure 3, puis alternativement un lien de mobilisation 5 situé sur un côté opposé de la deuxième portion 3.

On comprendra que de tels moyens mécaniques de mobilisation externe du dispositif 1 pourraient être complétés ou remplacés par des moyens alternatifs de mobilisation. Par exemple, des moyens de mobilisation électromécaniques et/ou électromagnétiques peuvent être utilisés. La deuxième portion 3 de boîtier peut par exemple être motorisée et contrôlable à distance.

Un avantage de l'utilisation de moyens de mobilisation électromécaniques et/ou électromagnétiques, associés par exemple à une unité de commande intégrée au dispositif 1 implantable, est de permettre le contrôle de la mobilisation externe du dispositif 1 à l'aide de signaux de commande, ce qui supprime la nécessité de tirer sur des liens de mobilisation au cours de la phase de mobilisation.

La mobilisation externe du dispositif 1 est de préférence contrôlée en temps réel par le praticien à l'aide de techniques d'imagerie médicale, par exemple par échographie, de sorte à surveiller la réalité des mouvements. De manière optionnelle, le dispositif 1 peut être équipé d'un ou plusieurs capteurs pour détecter un déplacement et/ou une vitesse et/ou une accélération de la deuxième portion 3 par rapport à la première portion 2. Le dispositif 1 comprend par exemple un ou plusieurs accéléromètres.

De préférence, le greffon de périoste reste vascularisé par un pédicule 44 (si on utilise un greffon vascularisé), notamment dans le cas où, au cours de la mobilisation externe du dispositif 1, la face inférieure de la portion 2 est immobilisée.

A ce titre, un orifice de passage 46 traversant est de préférence ménagé à travers la face inférieure de la première portion 2 de boîtier, de manière à laisser passer les vaisseaux du pédicule 44 du greffon de périoste. L'orifice de passage 46 traverse ici la paroi externe 24 de la première portion 2, comme représenté sur la Figure 4a. L'orifice de passage 46 est ici agencé dans une zone centrale de la grille 42 présente sur la première portion 2.

De préférence, l'orifice de passage 46 est de petite taille. En effet, le greffon de périoste pédiculé (qui est de préférence de faible épaisseur et souple) peut être enroulé transitoirement sur lui-même pour glisser au travers de l'orifice de passage 46 de petite taille. Le périoste vascularisé peut ensuite être déroulé et inséré dans l'espace de développement cellulaire.

En alternative ou en combinaison, un orifice de passage pourrait être prévu sur la deuxième portion 3 pour permettre le passage d'un pédicule de greffon de périoste. Cependant, il est avantageux que le pédicule 44 vasculaire passe à travers la première portion 2 qui reste immobile par rapport au support musculaire, pour éviter un pincement ou une torsion du pédicule 44 vasculaire du fait des mouvements de la deuxième portion 3 pendant la mobilisation.

La phase de mobilisation externe du dispositif 1 implantable peut être répétée à autant de reprises que nécessaire, pendant une durée suffisante pour générer des contraintes de cisaillement au sein du dispositif. Par exemple, une mobilisation de quelques minutes peut être effectuée quotidiennement, pendant une durée qui est par exemple comprise entre une minute et dix minutes.

A une étape ultérieure, une fois obtenus le ou les cartilages néoformés 12 dans l'espace de développement cellulaire à l'intérieur du dispositif 1, le dispositif 1 est extrait de l'organisme et démonté, de sorte à récupérer le ou les cartilages néoformés 12.

La région 80 intra-musculaire est réouverte pour extraire le dispositif 1 du muscle, puis on sépare la première portion 2 de la deuxième portion 3 (un exemple de démontage d'un dispositif 1 est décrit ci-après en relation à la Figure 8 annexée).

Comme illustré au bas de la Figure 3, le cartilage néoformé 12 obtenu comprend de préférence une surface cartilagineuse 120 (ici de forme convexe) en surface, et une zone d'os sous-chondral 122 néoformé sous-jacent à la surface cartilagineuse 120.

On a également représenté sur la Figure 3 une zone articulaire 9 à réparer, comprenant le secteur de cartilage endommagé 90.

Le cartilage néoformé 12 est ensuite implanté en lieu et place de du secteur lésionnel, au niveau de la cavité de parage 92, après parage de cette cavité. Dans le présent exemple, la zone articulaire 9 est un condyle fémoral. L'articulation traitée est une articulation fémoro-tibiale.

On comprendra toutefois que le cartilage néoformé pourrait également être implanté pour la réparation d'un secteur endommagé de cartilage situé dans une autre articulation. La pose d'un cartilage néoformé est notamment envisagée pour l'articulation fémoro-patellaire, l'articulation talo-crurale, l'articulation gléno-humérale, l'articulation acromio-claviculaire, l'articulation coxo-fémorale.

Le cartilage néoformé 12 obtenu est, de façon très avantageuse, adapté de manière précise à la géométrie du secteur de cartilage endommagé 90 à réparer. Ainsi, le cartilage néoformé 12 est conformé de manière précise au secteur de cartilage endommagé 90.

De préférence, on a préalablement réalisé une cavité de parage 92 sur la surface du secteur endommagé 90, le cartilage néoformé 12 étant alors implanté dans la cavité de parage 92.

Par « parage », on entend une manœuvre consistant à éroder le cartilage non sain et à mettre à nu l'os sous-chondral. Le parage permet de nettoyer le secteur endommagé de cartilage. De plus, le parage peut générer des micro-saignements, un afflux de néovaisseaux sanguins et une libération locale de facteurs de croissance au niveau de l'os sous-chondral natif.

La morphologie du cartilage néoformé 12 est contrôlée par le choix de la forme du dispositif 1 implantable. Par conception du dispositif 1, et notamment en fonction de la forme des parois délimitant l'espace de développement cellulaire, il est possible d'obtenir un cartilage adapté à une morphologie spécifique donnée de la cavité articulaire. A ce titre, la première portion 2 et/ou la deuxième portion 3 sont de préférence fabriquées sur mesure, en fonction de la morphologie du secteur endommagé 90 à réparer.

Une telle fabrication sur mesure est par exemple réalisée par impression 3D. On pourra utiliser toute technique d'impression 3D à l'aide de matériaux biocompatibles, par exemple une ou plusieurs des techniques décrites (en particulier à la page 22) dans la publication *Bioactive scaffolds for osteochondral regeneration,* Deng, Chang, Wu, Journal of Orthopaedic Translation (2019) 17, pp.15-25, l'impression par extrusion, l'impression laser, la stéréolithographie, le dépôt de fil fondu (FDM), le frittage sélectif laser (SLS), l'électro-filature, etc.

L'impression 3D du dispositif 1 implantable peut être basée sur un modèle tridimensionnel tenant compte de la géométrie du secteur de cartilage endommagé 90, obtenu par imagerie médicale.

Des exemples de techniques d'imagerie médicale utilisables à cette fin sont l'imagerie de résonance magnétique (IRM) (IRM en temps de relaxation T2 ou en T1RHO) et/ou l'imagerie IRM avec séquences d'acquisition dites « dGEMRIC » (delayed Gadolinium-Enhanced MRI of articular Cartilage) et/ou la radiographie standard et/ou l'arthroscanner.

Après avoir récupéré le ou les cartilages néoformés 12, on implante ledit cartilage sur la zone articulaire 9. La pose du cartilage néoformé 12 est éventuellement réalisée par arthroscopie ; une telle procédure est assez peu invasive.

Un avantage notable de l'approche proposée ici est de permettre une mise en œuvre du parage du secteur de cartilage endommagé 90 (ici de la surface du condyle fémoral) postérieure à l'obtention du cartilage néoformé 12.

Il est ainsi possible de contrôler la qualité et la forme de la portion du cartilage néoformé 12, avant parage de la zone lésionnelle du cartilage qui doit être réparé. On peut aussi adapter la forme de la cavité de parage 92 en fonction de la forme obtenue pour le cartilage néoformé 12. On peut aussi éventuellement renoncer au parage, dans la mesure où il s'agit d'un geste de destruction du cartilage natif encore en place (bien qu'endommagé), et à la procédure d'implantation, en cas de production insuffisante de cartilage néoformé au sein du dispositif.

Des exemples de dispositifs implantables, prévus pour favoriser la formation d'un nouveau cartilage, sont décrits ci-après en relation aux Figures 4a à 12 annexées. Des dispositifs implantables amovibles selon l'un quelconque de ces exemples peuvent être utilisés pour mettre en œuvre la procédure décrite ci-avant en relation à la Figure 3.

### Exemple 1A - Mobilisation en translation pour former deux portions de cartilage

La Figure 4a illustre schématiquement un dispositif 1 implantable amovible selon un premier exemple de réalisation, représenté en perspective du dessus. Le dispositif 1 est de préférence prévu pour être implanté de manière amovible dans une région intra-musculaire, et/ou prévu pour être implanté de manière amovible dans une région sous-cutanée.

Le dispositif 1 présente une forme générale de boîtier fermé. Le dispositif 1 comprend une première portion 2 de boîtier en matériau biocompatible et une deuxième portion 3 de boîtier en matériau biocompatible.

La première portion 2 est représentée sur une partie haute de la Figure 4a, et la deuxième portion 3 est représentée sur une partie basse de la Figure 4a. Sur cette Figure 4a, les deux portions de boîtier sont donc dissociées l'une de l'autre.

La première portion 2 et la deuxième portion 3 sont prévues pour définir entre elles une cavité, qui forme un espace 40 de développement cellulaire.

On a représenté sur la Figure 4b annexée le même dispositif 1, la première portion 2 de boîtier étant emboîtée avec la deuxième portion 3 de boîtier (par exemple *via* une glissière 6, qui n'a pas été représentée sur la Figure 4b pour plus de lisibilité de la figure). L'espace 40 de développement cellulaire est visible de côté, sur une partie droite de la Figure 4b.

L'intérieur du dispositif 1, prévu pour recevoir une matrice 4 cellulaire de réparation, est en outre visible en coupe transversale sur la Figure 5 annexée. Sur la vue en coupe transversale de cette Figure 5, et sur les Figures 7 et 9, l'écart vertical entre la face interne 26 de la première portion 2 et la face interne 36 opposée de la deuxième portion 3 n'est pas représenté à l'échelle, et a été agrandi pour une bonne lisibilité de ces figures. Ledit écart vertical est de dimension très faible (par exemple moins d'un millimètre) par rapport aux dimensions de la première portion 2 et de la deuxième portion 3.

L'espace 40 est prévu pour recevoir des cellules ostéochondrogéniques (notamment des cellules issues du développement de la matrice 4 cellulaire de réparation) et pour permettre une multiplication de ces cellules.

La deuxième portion 3 de boîtier est montée de manière amovible sur la première portion 2 de boîtier. La liaison de la deuxième portion 3 sur la première portion 2 est suffisamment résistante pour ne pas céder au cours de la sollicitation du dispositif 1 par les moyens de mobilisation externe (qui seront décrits ci-après) durant la phase de mobilisation.

La deuxième portion 3 est montée mobile sur la première portion 2 ; dans le présent exemple, la deuxième portion 3 est mobile en translation par rapport à la première portion 2 le long d'une direction parallèle à un axe longitudinal B du boîtier.

La première portion 2 et/ou la deuxième portion 3 sont formées de préférence dans un matériau biocompatible choisi parmi les matériaux polymères (tels que le PTFE), les matrices extracellulaires, les matériaux biocéramiques, les verres biologiques ou les métaux biocompatibles, ou dans un mélange quelconque de ces matériaux.

La première portion 2 de boîtier est au moins partiellement délimitée latéralement par une première face latérale 20 et une deuxième face latérale 22. Les faces latérales 20 et 22 sont sensiblement perpendiculaires à l'axe longitudinal du boîtier, et s'étendent le long de deux surfaces sensiblement parallèles.

Dans le présent exemple, la première face latérale 20 et la deuxième face latérale 22 comprennent chacune un creux central. Un canal creux est ainsi formé le long de l'axe longitudinal B de la première portion 2, reliant les faces latérales 20 et 22.

La première portion 2 de boîtier est en outre délimitée extérieurement par une face externe 24, et délimitée intérieurement par une face interne 26. La face externe 24 et la face interne 26 s'étendent sur deux surfaces sensiblement parallèles.

La face interne 26 est placée face à la deuxième portion 3 de boîtier. Dans le présent exemple, la face interne 26 est évidée en son centre (au niveau du canal creux entre les faces latérales 20 et 22) pour autoriser le développement des cellules ostéochondrogéniques jusqu'au fond de la première portion 2.

La première portion 2 de boîtier comprend en outre deux parois intérieures latérales 28 délimitant latéralement le canal creux. Les parois intérieures latérales 28 s'étendent en direction de la deuxième portion 3, à partir d'une paroi de fond située derrière la face externe 24.

La deuxième portion 3 de boîtier est délimitée latéralement par une première face latérale 30 et une deuxième face latérale 32. Les faces latérales 30 et 32 sont sensiblement perpendiculaires à l'axe longitudinal du boîtier et s'étendent sur deux surfaces sensiblement parallèles. Ici également, la première face latérale 30 et la deuxième face latérale 32 comprennent chacune un creux central. Un canal creux supplémentaire est ainsi formé le long de l'axe longitudinal B de la deuxième portion 3, reliant les faces latérales 30 et 32.

La deuxième portion 3 de boîtier est délimitée extérieurement par une face externe 34, et délimitée intérieurement par une face interne 36. La face externe 34 et la face interne 36 s'étendent sur deux surfaces sensiblement parallèles. La face interne 36 est placée face à la première portion 2 de boîtier.

La deuxième portion 3 de boîtier comprend en outre deux parois intérieures latérales 38 délimitant latéralement le canal creux. Les parois intérieures latérales 38 s'étendent en direction de la première portion 2, à partir d'une paroi de fond située derrière la face externe 34.

Dans le présent exemple et comme cela est représenté sur la Figure 4b, lorsque le boîtier constituant le dispositif 1 est fermé, les canaux creux respectifs de la première portion 2 et de la deuxième portion 3 sont placés l'un face à l'autre.

La Figure 6 annexée, qui est une vue en coupe longitudinale du dispositif 1, correspond à un plan de coupe passant par lesdits deux canaux creux. La Figure 6 illustre la première portion 2 et la deuxième portion 3 au cours de la mobilisation externe du dispositif 1. L'espace 40 de développement cellulaire s'étend à l'intérieur des deux canaux creux. Ledit espace 40 est délimité latéralement par les parois intérieures latérales 28 de la première portion 2, et par les parois intérieures latérales 38 de la deuxième portion 3. L'espace de développement cellulaire 40 est en outre délimité par le dessus (selon l'orientation de la Figure 4a par le côté intérieur de la face externe 34, et est délimité par le dessous par le côté intérieur de la face externe 24.

Ainsi, lorsque la deuxième portion 3 est alignée avec la première portion 2, l'espace de développement cellulaire 40 ménagé à l'intérieur du dispositif 1 présente une forme générale parallélépipédique.

La matrice de réparation 4 a pour fonction d'engendrer une multitude de cellules ostéochondrogéniques. La matrice de réparation 4 comprend avantageusement un greffon de périoste, dont la fonction biologique a été rappelée ci-avant.

Très avantageusement (mais non obligatoirement), le greffon de périoste est un greffon vascularisé, par exemple un greffon pédiculé vascularisé. La Figure 4a illustre un greffon de périoste attaché à un pédicule 44, le pédicule 44 traversant l'orifice de passage 46 prévu dans le corps de la première portion 2 de boîtier. On peut aussi envisager un greffon non vascularisé, ou éventuellement une combinaison de greffon non vascularisé et de greffon vascularisé dans le cas où au moins deux greffons différents sont utilisés.

Avantageusement, le greffon de périoste résulte d'une autogreffe. Autrement dit, les cellules formant le greffon de périoste sont des cellules autologues, issues du même individu chez lequel on pose l'implant articulaire. L'utilisation de cellules autologues favorise une bien meilleure tolérance des cellules ostéochondrogéniques, et à terme des chondrocytes du cartilage réparé, par l'organisme de l'individu. Ainsi, on promeut plus efficacement la régénération du cartilage et on limite fortement le risque de rejet.

Dans le présent exemple, le greffon périostique est issu d'un périoste de tibia. Un avantage est que le geste de prélèvement sur l'os du tibia est aisé et assez peu invasif.

De préférence, avant la sollicitation par les moyens de mobilisation externe, la matrice de réparation 4 revêt la forme d'un élément cohésif solide, non nécessairement solidaire du dispositif 1. Par exemple, la matrice de réparation 4 est insérée (et éventuellement suturée) dans l'espace de développement cellulaire 40 juste avant insertion du dispositif 1 dans la région intra-musculaire ou sous-cutanée.

Un avantage de l'orifice de passage 46 est de permettre la vascularisation de la matrice de réparation 4 tout au long de la phase de mobilisation par les moyens de mobilisation externe. L'orifice permet le passage d'au moins un vaisseau sanguin depuis l'extérieur de l'implant, pour que la matrice de réparation 4 soit vascularisée au cours de la mobilisation. Une telle vascularisation est très pertinente dans le cas où la matrice de réparation 4 comprend un greffon de périoste, afin de garantir un bon développement des cellules.

Ainsi, après pose de l'implant (par exemple dans la région intra-musculaire ou la région sous-cutanée), des cellules ostéochondrogéniques se multiplient *in vivo* à partir de la matrice de réparation 4.

On a représenté sur la vue de la Figure 6 les cellules ostéochondrogéniques remplissant l'espace 40 après leur multiplication à l'intérieur de l'implant. Dans le cas où la première portion 2 et/ou la deuxième portion 3 comprennent des pointes 43 coniques, les cellules ostéochondrogéniques remplissent préférentiellement les logettes définies par les pointes 43.

Dans le présent exemple, la deuxième portion 3 est configurée pour glisser par rapport à la première portion 2 le long de la face interne 26 au cours de son mouvement de translation, par une mobilisation externe du dispositif 1. On a représenté en trait pointillé sur la Figure 5 une surface P d'extension de la face interne 26.

Le dispositif 1 comprend des moyens de mobilisation externe, permettant de forcer le déplacement de la deuxième portion 3 par rapport à la première portion 2. Un cisaillement est ainsi généré entre la deuxième portion 3 et la première portion 2, notamment ici au voisinage de la surface P d'extension de la surface interne 26 de glissement. Des contraintes de cisaillement sont ainsi générées au sein de l'espace de développement cellulaire 40.

De telles contraintes de cisaillement ont pour avantage de diriger la différenciation des cellules présentes au voisinage de la surface P dans l'espace de développement cellulaire 40. En effet, les cellules les plus proches de la surface P sont les plus exposées aux contraintes de cisaillement. Les cellules vont donc se différencier préférentiellement vers des cellules de surface de cartilage (voie C de différenciation illustrée sur la Figure 2).

Ainsi, la mobilisation externe du dispositif 1 matérialise un plan de clivage à l'intérieur de l'espace de développement cellulaire 40, pour exercer des contraintes de cisaillement.

Les contraintes de cisaillement ainsi créées se rapprochent des contraintes exercées sur un plan de mobilité d'une articulation, ici entre un condyle fémoral et un plateau tibial.

On mobilise de préférence le dispositif 1 après que les cellules ostéochondrogéniques ont commencé à se multiplier (voir exemple illustré sur la Figure 6).

De préférence, les moyens de mobilisation externe comprennent des moyens mécaniques. De manière plus préférentielle, lesdits moyens comprennent au moins un lacet de mobilisation monté sur la deuxième portion 3, configuré pour permettre de tracter la deuxième portion 3 par rapport à la première portion 2.

On entend par « lacet de mobilisation » une attache (fil, câble, courroie...) fixée sur une partie du dispositif 1, pouvant être tirée pour forcer un mouvement relatif de la deuxième portion 3 par rapport à la première portion 2, ici un mouvement de translation. De préférence, ledit lacet de mobilisation est alors fixé sur une face extérieure, typiquement sur l'une des faces latérales 30 et/ou 32 de la deuxième portion 3.

Dans l'exemple des Figures 4a à 8, les moyens de mobilisation externe comprennent un premier lacet de mobilisation 5A fixé sur la face latérale 30 et un deuxième lacet de mobilisation 5B fixé sur la face latérale 32 opposée. Ainsi, les deux lacets de mobilisation 5A et 5B sont fixés sur des faces extérieures opposées de la deuxième portion 3. Ces liens n'ont pas été représentés sur la Figure 4b pour davantage de lisibilité.

Comme décrit précédemment en relation à la Figure 3, lorsque le dispositif 1 est implanté, les deux lacets de mobilisation 5A et 5B comportent de préférence une longueur interne de fil en sous-cutané et une longueur externe de fil destinée à demeurer à l'extérieur de la peau.

A l'aide des lacets de mobilisation 5A et 5B, la deuxième portion 3 peut être tractée du côté gauche puis du côté droit (selon l'orientation des Figures 4a et 6) par rapport à la première portion 2, de sorte à créer un cisaillement au sein de l'espace de développement cellulaire.

De préférence, les lacets de mobilisation 5A et 5B sont fabriqués avec des brins de fil semi-rigide. De préférence, lesdits lacets sont en matériau polymère. On peut par exemple utiliser tout matériau figurant usuellement dans des fils de suture.

De manière optionnelle et avantageuse, le dispositif 1 comprend en outre une glissière 6 guidant le déplacement de la deuxième portion 3 par rapport à la première portion 2. Ainsi, lors de la sollicitation du dispositif 1 par les moyens de mobilisation externe 5, la deuxième portion 3 glisse le long de la glissière 6, de sorte que la deuxième portion 3 ne se déplace pas dans un sens transversal par rapport à la première portion 2.

Une telle glissière est visible sur la Figure 4a ainsi que sur la Figure 5. Dans le présent exemple, la glissière 6 comporte au moins un monorail fixé sur la première portion 2. Le monorail est solidaire mécaniquement du reste de la première portion 2. Ledit monorail fait ici saillie de la face interne 26 orientée face à la deuxième portion 3. La deuxième portion 3 comporte alors, au niveau de sa face interne 36, au moins une rainure 60 complémentaire du monorail. Pour une bonne lisibilité des figures, la forme réelle de la rainure 60 n'est pas illustrée sur les Figures 4a et 4b.

De préférence, la glissière 6 présente deux monorails longitudinaux identiques des deux côtés, comme cela est représenté sur la Figure 4a.

En alternative, une glissière présentant un fonctionnement similaire pourrait être obtenue en disposant un ou deux monorails au niveau de la face interne 36 et une ou deux rainures complémentaires au niveau de la face interne 26.

De telles glissières 6 sont transposables à une variante du dispositif 1 où les moyens de mobilisation externe seraient électriques et non mécaniques.

Par ailleurs, le dispositif 1 comprend optionnellement des moyens pour fixer la première portion 2 par rapport à l'organisme, par exemple par rapport à la région intra-musculaire ou sous-cutanée dans laquelle le dispositif 1 est présent.

A ce titre, le dispositif 1 comprend de préférence au moins un lien de fixation, encore plus préférentiellement une paire de liens de fixation. Sur la Figure 4a, le dispositif 1 comprend un premier lien de fixation 5C sur la face latérale 30, et comprend en outre un deuxième lien de fixation 5D sur la face latérale 32. Ces liens n'ont pas été représentés sur la Figure 4b pour davantage de lisibilité.

Le lien de fixation 5C et/ou le lien de fixation 5D sont par exemple attachés à un muscle 8 dans lequel le dispositif 1 est implanté. Les liens de fixation 5C et 5D sont par exemple suturés au muscle 8, de sorte que la première portion 2 demeure fixe pendant que la deuxième portion 3 est déplacée en translation au cours de la phase de mobilisation externe. En alternative, les liens de fixation 5C et 5D peuvent présenter des longueurs externes de fil respectives destinées à demeurer à l'extérieur de la peau, à l'image des lacets de mobilisation 5A et 5B. Les liens de fixation 5C et 5D peuvent alors être immobilisés, par exemple manuellement, au niveau de ces longueurs externes de fil.

De préférence, les liens de fixation 5C et 5D sont fabriqués avec des brins de fil semi-rigide. De préférence, lesdits liens sont en matériau polymère. On peut par exemple utiliser tout matériau figurant usuellement dans des fils de suture.

De préférence, la première portion 2 et/ou la deuxième portion 3 comprennent une structure creuse, formant des logements creux qui prolongent l'espace de développement cellulaire 40.

Dans l'exemple des Figures 4a à 8, la première portion 2 présente une grille 42 formant une telle structure creuse.

La grille 42 s'étend depuis l'intérieur de la face externe 24, selon une direction d'extension (largeur) perpendiculaire à la face externe 24. Pour une bonne lisibilité des figures annexées, la grille 42 n'a pas été illustrée sur les Figures 4a et 4b.

La grille 42 définit, dans le sens de la largeur (direction d'extension perpendiculaire à la face externe 24), une pluralité de cavités de croissance cellulaire traversantes débouchant d'un côté intérieur du dispositif 1.

Les cavités de croissance cellulaire sont ici définies entre des pointes 43 coniques formées dans la grille 42. De préférence, une base des pointes 43 coniques est orientée vers l'extérieur et un sommet des pointes 43 coniques est orienté vers l'intérieur.

Des logettes creuses (par exemple des logettes parallélépipédiques, et/ou alvéolaires) traversantes sont formées entre les pointes 43 coniques. Les espaces intérieurs des logettes se trouvent dans le prolongement de la zone où la matrice de réparation 4 est placée. Ainsi, les espaces intérieurs des logettes prolongent l'espace de développement cellulaire 40. La matrice de réparation 4 peut alors être posée et/ou fixée au-dessus d'une surface supérieure de la grille 42 de la première portion 2, comme illustré sur la Figure 5.

Les cavités de croissance (ici les logettes à l'intérieur de la grille 42) sont ici traversantes des deux côtés. Du côté extérieur, les logettes traversent la face externe 24 de la première portion 2, et du côté intérieur, les logettes rejoignent l'espace de développement cellulaire 40.

Un avantage d'une telle grille 42 est qu'au cours du mouvement de translation de la deuxième portion 3 par rapport à la première portion 2, les cellules situées au voisinage de la face externe 24 (qui sont les cellules les plus éloignées de la surface P de clivage) sont plus stables mécaniquement que les cellules au voisinage de la surface P de clivage.

Au cours de la mobilisation externe du dispositif implantable, un gradient de rigidité contre le cisaillement est ainsi créé à l'intérieur de l'espace de développement cellulaire 40. Les cellules situées au voisinage de la face externe 24 sont amenées à former préférentiellement des cellules d'os après différenciation (voie A de la Figure 2). Il est ainsi possible d'obtenir des couches d'os sous-chondral dans le cartilage néoformé 12 obtenu à l'issue de la mobilisation du dispositif 1.

De plus, la grille 42 permet à l'irrigation sanguine de parvenir à l'intérieur de l'espace de développement cellulaire 40, depuis l'extérieur du dispositif 1.

En outre, un avantage de l'utilisation de pointes 43 coniques pour former la grille 42 est que les parois séparatrices de la grille 42 présentent alors une épaisseur au niveau de la base supérieure à leur épaisseur au niveau de la surface, ce qui accentue le gradient de rigidité contre le cisaillement.

Pour obtenir la grille 42, une ou plusieurs cavités peuvent être prévues nativement dans la conception de la première portion 2. Par exemple, un modèle 3D de la première portion 2 peut intégrer les cavités de la grille 42 dès sa conception. Alternativement, les cavités de la grille 42 peuvent être obtenues par perforation, après la fabrication.

Un avantage additionnel de fournir une grille 42 au niveau de la première portion 2 est de permettre une intercommunication entre la matrice de réparation 4 et l'environnement de la région intra-musculaire, et notamment une meilleure vascularisation de la matrice de réparation 4.

Dans le présent Exemple 1A, la deuxième portion 3 ne comprend pas de grille. Toutefois, la deuxième portion 3 comprend ici des pointes 43 coniques formant des cavités de croissance dans le prolongement de l'espace de développement cellulaire 40. Ainsi, les pointes coniques de la deuxième portion 3 sont en regard des pointes coniques de la première portion 2. A la différence de la première portion 2, les espaces entre les pointes 43 coniques de la deuxième portion 3 ne forment pas des perforations traversantes.

De manière alternative, la deuxième portion 3 pourrait être également dotée de perforations traversantes, par exemple à l'image de la grille 42. Toutefois, il est avantageux que la face externe 34 ne soit pas perforée et que les cavités formées entre les pointes 43 coniques de la deuxième portion 3 ne soient pas traversantes. En effet, la deuxième portion 3 est amenée à être déplacée par rapport à la région musculaire. La face externe 34 lisse permet d'éviter des frictions ou frottements entre la deuxième portion 3 et l'environnement musculaire au cours du déplacement de la deuxième portion 3.

De manière optionnelle et avantageuse, au niveau de leur base, au moins certaines des pointes 43 coniques de la première portion 2 et/ou de la deuxième portion 3 peuvent présenter des évidements. De préférence, la forme des évidements des pointes 43 coniques est sélectionnée pour ne pas gêner l'extraction ultérieure du tissu ostéo-cartilagineux néoformé.

A titre d'exemple, un tel évidement peut présenter une forme triangulaire au niveau de la base de la pointe 43 conique, et s'étendre selon une forme pyramidale en direction du sommet du cône (par exemple, selon une forme de pyramide à trois côtés). De préférence, l'évidement triangulaire ainsi réalisé dans la surface de la pointe 43 conique ne s'étend pas jusqu'au sommet de ladite pointe 43.

De telles pointes 43 coniques évidées sont avantageuses, car elles agrandissent la surface totale de contact entre les cellules ostéochondrogéniques formées et le support en biomatériel à l'intérieur de l'espace de développement cellulaire. De plus, la rigidité de la première portion 2 de support et/ou de la deuxième portion 3 de support est localement accrue. Pour simplifier les figures annexées, les pointes 43 coniques n'ont pas été représentées avec de tels évidements sur ces figures.

En alternative, les pointes 43 coniques du dispositif 1 ne présentent pas d'évidements.

De manière préférentielle, des moyens de blocage en translation sont prévus sur la première portion 2 et/ou sur la deuxième portion 3. Les moyens de blocage en translation permettent de définir la plage de déplacement en translation de la deuxième portion 3 par rapport à la première portion 2.

Dans le présent exemple, de tels moyens de blocage en translation comprennent au moins une protubérance 27 configurée pour buter contre au moins une cale 37.

Ici, la première portion 2 comprend une paire de protubérances 27 faisant saillie vers le haut, sur un côté de la première portion 2. Les protubérances 27 sont de préférence placées axialement proches l'une de l'autre le long de l'axe longitudinal B, sur un côté de l'axe longitudinal B. Chaque protubérance 27 comprend par exemple une base, et une tige faisant saillie de la base avec une terminaison sphérique.

La deuxième portion 3 comprend ici, quant à elle, une paire de cales 37. Les cales 37 sont placées l'une face à l'autre, sur le même côté de l'axe longitudinal B. De préférence, la distance entre les faces internes des cales 37 le long de l'axe longitudinal B est strictement supérieure à la distance entre les protubérances 27 le long de l'axe longitudinal B.

Ainsi, lorsque la première portion 2 et la deuxième portion 3 ont été emboîtées comme illustré sur la Figure 4b, les protubérances 27 sont insérées dans leurs bases sur la face latérale de l'élément 2 à l'intérieur de l'espace longitudinal ménagé entre les cales 37. La plage de déplacement en translation de la deuxième portion 3 par rapport à la première portion 2 est alors limitée par la mise en butée des protubérances 27 sur les faces internes des cales 37.

On comprendra que de manière alternative, les cales et protubérances peuvent être disposées de l'autre côté, ou des deux côtés, et/ou une ou plusieurs protubérances peuvent être fournies sur la deuxième portion 3, et/ou une ou plusieurs cales peuvent être fournies sur la première portion 2.

A l'issue de la mobilisation du dispositif 1 par les moyens de mobilisation externe, qui peut éventuellement être répétée de manière régulière pendant une phase de mobilisation, les cellules ostéochondrogéniques de l'espace 40 se différencient en cellules de cartilage et d'os sous-chondral. Ainsi, dans l'Exemple 1A on obtient deux portions en regard de cartilage néoformé 12 en regard l'une de l'autre, comme illustré sur la Figure 7 annexée.

A la surface de la grille 42, les cellules les plus proches de la surface P se sont préférentiellement différenciées en cellules de cartilage, pour obtenir des surfaces cartilagineuses 120.

A la base de la grille 42 (au voisinage des faces externes 24 et 34 des deux côtés du boîtier), les cellules les plus éloignées de la surface P se sont préférentiellement différenciées en cellules d'os sous-chondral, pour obtenir des zones sous-chondrales 122.

On a représenté sur la Figure 8 un démontage du dispositif 1 après extraction dudit dispositif par rapport à la région sous-cutanée ou intra-musculaire. Dans le présent exemple, on exerce un effort mécanique pour séparer la première portion 2 et la deuxième portion 3. On rompt ainsi la glissière 6 de préférence au niveau du monorail. Par exemple, une base du monorail reste sur la première portion 2 et une partie distale du monorail est emportée par la deuxième portion 3.

On obtient ainsi deux cartilages intègres, prêts à être assimilés dans la cavité articulaire afin de réparer les secteurs endommagés de cartilage.

Dans le présent exemple, les secteurs endommagés de cartilage se situent dans une articulation fémoro-tibiale. Par exemple, l'un des cartilages néoformés 12 obtenus est positionné au niveau d'un condyle fémoral, et l'autre des cartilages néoformés 12 est positionné au niveau d'un plateau tibial faisant face au condyle fémoral. Chaque cartilage néoformé est préférentiellement enchâssé directement dans le secteur endommagé correspondant, par exemple dans une cavité de parage respective.

De manière préférentielle, les cartilages néoformés 12 obtenus se conforment aux géométries des secteurs endommagés de cartilage à réparer, et le cas échéant, se conforment aux géométries des cavités de parage. Après pose du cartilage néoformé 12, la surface du cartilage s'étend préférentiellement dans la continuité des surfaces voisines au secteur endommagé de cartilage.

Les étapes de mobilisation et dissociation des portions de dispositif implantable telles que décrites ci-avant, pour l'exemple des Figures 4a à 8 annexées, se transposent de manière similaire aux autres exemples de dispositifs implantables amovibles décrits ci-après en relation aux Figures 9 à 12 annexées.

Un avantage d'un dispositif implantable selon le premier exemple décrit ci-avant est de permettre la formation simultanée de deux secteurs de cartilage néoformé 12, adaptés pour implantation dans deux secteurs endommagés de cartilage appartenant préférentiellement à deux surfaces en regard d'une même articulation.

L'épaisseur totale moyenne des cartilages d'une articulation fémoro-tibiale humaine adulte est de 5 millimètres environ. Ainsi, une épaisseur moyenne du cartilage néoformé 12 (perpendiculairement à une surface d'extension de la face extérieure du cartilage) est par exemple comprise entre 1 millimètre et 10 millimètres.

### Exemple 1B - Mobilisation en translation avec logement unique de formation de cartilage

La Figure 9 illustre schématiquement un dispositif implantable selon un deuxième exemple de réalisation. Le dispositif implantable est vu en coupe transversale, perpendiculairement à un axe d'extension longitudinale.

Le dispositif implantable selon ce deuxième exemple présente des caractéristiques structurelles et fonctionnelles très proches du dispositif 1 selon le premier exemple décrit ci-avant, à l'exception de la structure de la première portion 2 de boîtier. La deuxième portion 3 de boîtier est mobile en translation par rapport à la première portion 2 de boîtier.

A la différence de l'Exemple 1A précédent, la première portion 2 (qui est de préférence maintenue fixe par rapport à la région intra-musculaire) n'est pas perforée et ne comprend pas de grille 42. La première portion 2 comprend ici une face interne 26' pleine, en regard de la deuxième portion 3. Dans le cas où une glissière 6 est incluse, la face interne 26' peut s'étendre entre deux monorails longitudinaux de la glissière 6.

De manière optionnelle et avantageuse, un réseau d'éléments de rigidification 7 est disposé à l'intérieur de la première portion 2. Les éléments de rigidification 7 sont placés du côté intérieur de la première portion 2, contre la face interne 26'. Le réseau d'éléments de rigidification 7 recouvre de préférence une majeure partie de l'étendue de la face interne 26'.

Les éléments de rigidification 7 présentent par exemple une forme de croisillons, et sont de préférence fixés sur la face interne 26'. Une forme de croisillons est par exemple obtenue par impression 3D de biomatériel. De tels éléments de rigidification 7 sont représentés sur la Figure 9.

Un avantage des éléments de rigidification 7 est d'augmenter la rigidité contre le cisaillement au fond de l'espace de développement cellulaire 40, du côté de la première portion 2. On accroît ainsi le gradient de rigidité entre la première portion 2 et la deuxième portion 3.

De préférence, dans ce deuxième exemple, le greffon de périoste reste vascularisé par un pédicule. A ce titre, un orifice de passage traversant est de préférence ménagé à travers la face inférieure de la première portion 2, afin de laisser passer les vaisseaux du pédicule.

La deuxième portion 3 est mobile en translation par rapport à la première portion 2. Par exemple, une glissière 6 est fixée solidairement à l'intérieur de la première portion 2, pour guider le mouvement le long de la surface P comprenant la face interne 26'. Au cours de la phase de mobilisation du dispositif implantable, les cellules les plus proches de la surface P tendent à subir un cisaillement, tandis que les cellules au fond des pointes coniques 43 tendent à se déplacer avec la deuxième portion 3. Les cellules les plus éloignées de la surface P se différencient préférentiellement en os sous-chondral.

Après mobilisation et développement cellulaire, un tissu ostéo-cartilagineux néoformé complet, comprenant une surface de cartilage et une région d'os sous-chondral, est typiquement obtenu à l'intérieur de la première portion 2 et permet de traiter le secteur articulaire endommagé. Après extraction du dispositif implantable selon cet Exemple 1B par rapport à l'organisme, la première portion 2 et la deuxième portion 3 sont dissociées du tissu ostéo-cartilagineux. Les pointes 43 coniques ne sont pas intégrées au tissu ostéo-cartilagineux néoformé.

Le cartilage éventuellement généré du côté de la deuxième portion 3, dans lequel sont incrustés les éléments de rigidification 7, n'est ici pas réutilisé chez le patient.

Dans une variante possible, des éléments de rigidification 7 pourraient être positionnés du côté de la deuxième portion 3 mobile (contre la face 34), en remplacement ou en combinaison avec les pointes 43 coniques. Les éléments de rigidification 7 sont alors optionnellement dissociés de la deuxième portion 3 lors de l'extraction du tissu ostéo-cartilagineux néoformé, et restent intégrés à ce tissu néoformé. Une intégration des éléments de rigidification 7 dans le cartilage à implanter est notamment envisageable si les éléments de rigidification 7 sont formés dans un biomatériel biorésorbable, ou dans un biomatériel très bien toléré sur le long terme à l'intérieur de l'articulation traitée.

### Exemple 2A - Mobilisation en rotation pour former deux portions de cartilage

La Figure 10 illustre schématiquement un dispositif implantable selon un troisième exemple de réalisation. Ledit dispositif est de préférence prévu pour être implanté de manière amovible dans une région intra-musculaire d'un individu, et/ou prévu pour être implanté dans une région sous-cutanée d'un individu. La Figure 10 représente le dispositif implanté à l'intérieur d'un muscle 8, sous l'épiderme 82, vu en coupe longitudinale.

Ce troisième exemple diffère des exemples décrits ci-avant principalement par le mouvement rotatif de la deuxième portion 3 de boîtier par rapport à la première portion 2 de boîtier, et par la structure de la première portion 2 et de la deuxième portion 3.

La première portion 2 présente ici une forme générale cylindrique autour de l'axe R, ouverte vers le bas (selon l'orientation de la Figure 10). La première portion 2 comprend une paroi latérale 28 et une paroi de fond 29. La paroi de fond 29 est de préférence en forme de disque. La paroi latérale 28 s'étend vers le bas à partir d'un bord annulaire de la paroi de fond 29.

La deuxième portion 3 présente également une forme générale cylindrique autour de l'axe R, ouverte vers le haut (selon l'orientation de la Figure 10). La deuxième portion 3 comprend une paroi latérale 38 et une paroi de fond 39. La paroi de fond 39 est en forme de disque. La paroi latérale 38 s'étend vers le haut à partir d'un bord annulaire de la paroi de fond 39.

La deuxième portion 3 est montée de manière amovible sur la première portion 2.

De préférence, la première portion 2 et/ou la deuxième portion 3 présentent une symétrie en rotation autour de l'axe R. La première portion 2 et la deuxième portion 3 refermées l'une sur l'autre forment ainsi un boîtier fermé de forme générale cylindrique.

Le diamètre externe de la paroi latérale 38 est avantageusement inférieur au diamètre interne de la paroi latérale 28, de sorte que la deuxième portion 3 est mobile en rotation autour de l'axe R à l'intérieur de la paroi latérale 38.

De manière similaire aux deux exemples précédents, la deuxième portion 3 est configurée pour être déplacée par rapport à la première portion 2, notamment sous l'effet d'une mobilisation externe.

De préférence, le dispositif comprend une glissière 6 prévue pour guider la rotation de la deuxième portion 3 autour de l'axe R par rapport à la première portion 2.

Dans l'exemple des Figures 10 et 11, la glissière 6 comprend une nervure annulaire s'étendant radialement du côté extérieur (par rapport à l'axe R) à partir de la paroi latérale 38 de la deuxième portion 3, de préférence au voisinage d'un bord libre de la paroi latérale 38. La première portion 2 comprend alors une rainure annulaire orientée radialement vers l'intérieur (par rapport à l'axe R) à partir de la paroi latérale 28. On comprendra que, de façon alternative, une glissière peut être formée par une nervure annulaire de la première portion 2 dirigée vers l'intérieur, complémentaire à une rainure de la deuxième portion 3.

De façon optionnelle, le dispositif comprend des moyens additionnels de fixation et de guidage en rotation de la deuxième portion 3 par rapport à la première portion 2.

A titre d'exemple, le bord libre de la paroi latérale 28 (dirigé vers la deuxième portion 3) peut présenter des filetages externes sur le côté radialement extérieur. Lors du montage de la deuxième portion 3 sur la première portion 2, pour obtenir le boîtier fermé, un anneau de fixation supplémentaire de diamètre supérieur au diamètre extérieur de la première portion 2 peut être rapporté par-dessus la glissière 6 et se refermer sur le bord libre de la paroi latérale 28. L'anneau de fixation présente alors préférentiellement, du côté radialement intérieur, des filetages internes complémentaires avec les filetages externes de la paroi latérale 28, permettant de visser ledit anneau contre ladite paroi. Un tel anneau de fixation n'est pas représenté sur les Figures 10 à 12.

Un espace de développement cellulaire 40 est défini axialement entre le côté intérieur de la paroi de fond 29 et le côté intérieur de la paroi de fond 39. Cet espace de développement cellulaire 40 peut recevoir une matrice de réparation 4. Cette matrice présente les caractéristiques déjà décrites ci-avant. Ladite matrice comprend notamment de préférence un greffon de périoste. De préférence, là encore dans ce troisième exemple, le greffon de périoste reste vascularisé par un pédicule. A ce titre, la première portion 2 et/ou la deuxième portion 3 peut comprendre un orifice de passage du pédicule (non représenté sur les figures annexées).

Comme dans les exemples précédents, la matrice de réparation 4 a pour fonction d'engendrer une multitude de cellules ostéochondrogéniques dans l'espace de développement cellulaire 40. De préférence, après un certain temps de développement cellulaire, les cellules ostéochondrogéniques comblent l'espace intérieur de la première portion 2 et l'espace intérieur de la deuxième portion 3, comme illustré sur la Figure 11.

De manière similaire aux deux exemples précédents, des moyens de mobilisation externe 5 sont actionnables pour déplacer la deuxième portion 3 par rapport à la première portion 2, de sorte à générer un cisaillement au sein de l'espace de développement cellulaire 40.

Dans le présent exemple, les moyens de mobilisation externe 5 comprennent une poignée mobile en rotation autour de l'axe de rotation R. La poignée mobile est ici fixée à la paroi de fond 39. La poignée mobile comprend ici un manche 54 s'étendant à partir de la paroi de fond 39 le long de l'axe R et une partie de préhension 56 (ici en forme de volant) s'étendant vers l'extérieur à partir du manche 54.

La poignée mobile est solidaire de la deuxième portion 3 de boîtier, dans sa rotation autour de l'axe R. Ainsi, si un praticien ou si le patient saisit la partie de préhension 56 et pivote ladite partie comme illustré sur la Figure 11, la deuxième portion 3 est entraînée en rotation.

De préférence, pendant la sollicitation mécanique de la deuxième portion 3, la première portion 2 demeure fixe par rapport au muscle 8. Des moyens de stabilisation de la première portion 2 sont par exemple prévus à cet effet, tels que des attaches 83.

Les cellules ostéochondrogéniques présentes dans le volume situé radialement à l'intérieur de la paroi latérale 38 tendent à être entraînées par le mouvement rotatif de la deuxième portion 3, tandis que les cellules ostéochondrogéniques présentes dans le volume situé radialement à l'intérieur de la paroi latérale 28 tendent à rester fixes.

On a représenté une surface de cisaillement Z, à l'interface entre l'espace à l'intérieur de la paroi latérale 28 et l'espace à l'intérieur de la paroi latérale 38. Au cours du mouvement rotatif de la deuxième portion 3, des contraintes de cisaillement sont générées notamment au niveau des cellules situées au voisinage de la surface de cisaillement Z.

Comme décrit ci-avant, de telles contraintes de cisaillement favorisent une différenciation dirigée des cellules ostéochondrogéniques vers des cellules de cartilage. On favorise le développement de surfaces cartilagineuses près de la surface de cisaillement Z.

De manière optionnelle et avantageuse, pour créer un gradient de rigidité contre le cisaillement à l'intérieur de l'espace 40 (rigidité élevée contre le cisaillement au voisinage des parois de fond 29 et 39, et rigidité plus faible près de la surface de cisaillement Z), la première portion 2 de boîtier et/ou la deuxième portion 3 de boîtier comprennent des pointes 43 coniques s'étendant à partir des parois de fond respectives 29 et 39 des deux dites portions de boîtier. Les pointes 43 coniques forment entre elles des logettes, par exemple de forme parallélépipédique ou de forme alvéolaire. L'espace de développement cellulaire 40 se prolonge jusqu'au fond des logettes.

Dans le présent exemple, des pointes 43 coniques sont fournies à la fois dans la première portion 2 et dans la deuxième portion 3. Un avantage est d'augmenter le gradient de rigidité contre le cisaillement à l'intérieur de l'espace de développement cellulaire 40. De plus, les pointes 43 coniques forment des cavités qui prolongent l'espace de développement cellulaire 40 entre la première portion 2 et la deuxième portion 3. On favorise ainsi la formation d'une zone sous-chondrale dans le cartilage néoformé obtenu.

Sur la première portion 2 fixe, les cavités formées entre les pointes 43 coniques sont de préférence des cavités traversantes. Ainsi, une grille 42 est formée sur la paroi de fond 29, qui est amenée à demeurer fixe.

En revanche, la paroi de fond 39 n'est de préférence pas perforée. La surface externe de la paroi de fond 39 est de préférence lisse.

De manière optionnelle et avantageuse, le dispositif implantable comprend en outre des moyens de stabilisation de la première portion 2 par rapport à la zone de l'organisme dans laquelle le dispositif est implanté, ici le muscle 8. Dans cet exemple, le dispositif comprend des attaches 83. Les attaches 83 relient fixement la première portion 2 (par exemple la paroi latérale 28) à l'épiderme 82. Les attaches 83 comprennent par exemple deux fils de suture fixés chacun à une extrémité sur la paroi latérale 28, et à l'extrémité opposée sur l'épiderme 82. Ainsi, au cours de la mobilisation de la deuxième portion 3, la première portion 2 demeure fixe par rapport au muscle 8.

Un avantage d'un dispositif implantable selon ce troisième exemple est que la première portion 2 et la deuxième portion 3 ne sont pas déplacées axialement l'une par rapport à l'autre au cours de la mobilisation. Les deux volumes de cellules ostéochondrogéniques compris respectivement à l'intérieur de la première portion 2 et de la deuxième portion 3 demeurent en contact tout au long de la mobilisation. Cela permet d'éviter que les contraintes de cisaillement soient insuffisantes au niveau des bords.

Le dispositif implantable selon ce troisième exemple permet la formation simultanée de deux cartilages. Après extraction des tissus ostéo-cartilagineux néoformés, les pointes 43 coniques (si présentes) ne sont préférentiellement pas intégrées aux tissus néoformés.

Dans une variante possible, le dispositif implantable de cet Exemple 2B peut comprendre un ou plusieurs réseaux d'éléments de rigidification 7 à l'intérieur du boîtier. Les éléments de rigidification 7 de structure similaire à celle décrite ci-avant sont par exemple positionnés contre la paroi de fond 29 et/ou contre la paroi de fond 39, et présentent de préférence une forme de croisillons. De préférence, les éléments de rigidification 7 viennent en remplacement des pointes 43 coniques et recouvrent une majeure partie de l'étendue de la paroi de fond correspondante. De tels éléments de rigidification 7 peuvent être intégrés aux tissus ostéo-cartilagineux néoformés (notamment si ces éléments de rigidification sont formés en matériau biorésorbable ou dans un autre matériel bien toléré à long terme par l'articulation).

### Exemple 2B - Mobilisation en rotation pour former un cartilage de forme convexe

On a représenté sur la Figure 12 un dispositif implantable selon un quatrième exemple, similaire structurellement et fonctionnellement au troisième exemple.

Dans ce quatrième exemple, la deuxième portion 3 est mobile en rotation autour de l'axe R par rapport à la première portion 2, *via* des moyens de mobilisation externe 5. Le dispositif du quatrième exemple diffère du dispositif du troisième exemple par certaines caractéristiques structurelles de la première portion 2 et de la deuxième portion 3. Pour le montage de la deuxième portion 3 sur la première portion 2, on peut prévoir, comme dans l'exemple précédent, un anneau de fixation fileté ajustable sur la deuxième portion 3.

Dans ce quatrième exemple, la paroi de fond 39 de la deuxième portion 3 est de préférence lisse et non perforée. La paroi de fond 29 de la première portion 2 est quant à elle de préférence perforée, de sorte à former une grille 42 favorisant la vascularisation de la matrice de réparation 4.

De préférence, dans ce quatrième exemple, le greffon de périoste reste vascularisé par un pédicule. A ce titre, un orifice de passage traversant est de préférence ménagé à travers la première portion 2, afin de laisser passer les vaisseaux du pédicule.

La paroi de fond 29 de la première portion 2 et/ou la paroi de fond 39 de la deuxième portion 3 ne présentent pas ici une géométrie plane. Les deux parois de fond 29 et 39 sont ici incurvées. De préférence, une convexité des parois de fond 29 et 39 correspond à une convexité du secteur endommagé de cartilage à réparer. La convexité nécessaire pour la forme des parois de fond 29 et 39 est déterminée par exemple à partir d'un modèle 3D d'un secteur de cartilage à traiter. Un avantage est de prévoir, dès la conception du dispositif implantable, une forme finale appropriée pour le cartilage néoformé obtenu à l'issue de la phase de mobilisation.

On comprendra qu'une telle adaptation de la convexité des portions mobiles est également applicable pour l'un quelconque des autres exemples décrits ci-avant.

En outre, le dispositif implantable de cet Exemple 2B comprend ici des éléments de rigidification 7 positionnés contre la paroi de fond 39. De préférence, les éléments de rigidification 7 viennent en remplacement des pointes 43 coniques de la deuxième portion 3 de l'Exemple 2A précédent. Un dispositif implantable selon le quatrième exemple est adapté pour permettre la formation d'un unique cartilage néoformé du côté de la première portion 2, pour traiter un unique secteur endommagé de cartilage ayant par exemple une forme non plate (de forme convexe dans le présent Exemple 2B).

## Revendications

1. Dispositif implantable amovible destiné à la production de cartilage articulaire, comprenant :
• une première partie (2) de support en matériau biocompatible,
• une deuxième partie (3) de support en matériau biocompatible montée mobile sur la première partie (2) de support, la première partie (2) de support et la deuxième partie (3) de support définissant entre elles une cavité formant un espace de développement cellulaire (40),
l'espace de développement cellulaire (40) étant prévu pour recevoir des cellules ostéochondrogéniques se multipliant dans l'espace de développement cellulaire (40),
• des moyens de mobilisation externe (5A, 5B) actionnables configurés pour déplacer la deuxième partie (3) de support par rapport à la première partie (2) de support, de sorte à générer un cisaillement au sein de l'espace de développement cellulaire (40).

2. Dispositif selon la revendication 1, dans lequel au moins l'une parmi la première partie (2) de support et la deuxième partie de support comprend une paroi de fond de support et comprend deux parois latérales de support (28) s'étendant à partir de la paroi de fond de support, lesdites deux parois latérales de support (28) délimitant l'espace de développement cellulaire (40).

3. Dispositif selon l'une quelconque des revendications 1 ou 2, dans lequel le dispositif comprend un boîtier fermé, formé par la première partie (2) de support et par la deuxième partie (3) de support, l'espace de développement cellulaire (40) étant défini à l'intérieur du boîtier.

4. Dispositif selon l'une quelconque des revendications 1 à 3, dans lequel la première partie (2) de support comprend une face interne (26) faisant face à la deuxième partie (3) de support, la deuxième partie (3) de support étant configurée pour glisser par rapport à la première partie (2) de support le long de la face interne (26), de préférence dans lequel la deuxième partie (3) de support est montée mobile en translation par rapport à la première partie (2) de support, le long d'une direction parallèle à une surface d'extension (P) de la face interne (26).

5. Dispositif selon l'une quelconque des revendications 1 à 4, dans lequel la deuxième partie (3) de support est montée mobile en rotation par rapport à la première partie (2) de support autour d'un axe de rotation (R).

6. Dispositif selon la revendication 5, dans lequel au moins l'une parmi la première partie de support et/ou la deuxième partie de support (3) comprend une face latérale (38) de forme cylindrique s'étendant autour de l'axe de rotation (R), de préférence dans lequel ladite partie (3) de support comprend en outre une base (39) sensiblement perpendiculaire à la face latérale (38), les moyens de mobilisation externe (5) comprenant une poignée (54) solidaire en rotation avec la base (39) et mobile en rotation autour de l'axe de rotation (R).

7. Dispositif selon l'une quelconque des revendications 1 à 6, dans lequel les moyens de mobilisation externe comprennent au moins un lacet de mobilisation (5A) monté sur la deuxième partie (3) de support, configuré pour tracter la deuxième partie (3) de support.

8. Dispositif selon la revendication 7, dans lequel le lacet de mobilisation (5A) est monté sur une première face externe (30) de la deuxième partie (3) de support, le dispositif comprenant en outre un lien de mobilisation supplémentaire (5B) monté sur une deuxième face externe (32) de la deuxième partie (3) de support, la deuxième face externe (32) étant opposée à la première face externe (30).

9. Dispositif selon l'une quelconque des revendications 7 ou 8, dans lequel le lacet de mobilisation (5A, 5B) comprend un brin semi-rigide, de préférence un brin en matériau polymère.

10. Dispositif selon l'une quelconque des revendications 1 à 9, le dispositif comprenant une glissière (6) configurée pour guider un déplacement de la deuxième partie (3) de support par rapport à la première partie (2) de support.

11. Dispositif selon l'une quelconque des revendications 1 à 10, dans lequel au moins l'une parmi la première partie (2) de support et/ou la deuxième partie (3) de support comprend une grille (42), la grille (42) définissant une pluralité de cavités de croissance cellulaire débouchant d'un côté intérieur du dispositif.

12. Dispositif selon l'une quelconque des revendications 1 à 11, dans lequel au moins l'une parmi la première partie (2) de support et/ou la deuxième partie (3) de support comprend un réseau d'éléments de rigidification (7) fixés à ladite partie de support, une rigidité contre le cisaillement au niveau des éléments de rigidification (7) étant strictement supérieure à une rigidité contre le cisaillement au niveau d'une zone centrale de l'espace de développement cellulaire (40).

13. Implant pour la formation de cellules de cartilage, l'implant comprenant :
• un dispositif implantable (1) selon l'une quelconque des revendications 1 à 12,
• une matrice (4) cellulaire de réparation, intercalée entre la première partie (2) de support du dispositif implantable et la deuxième partie (3) de support du dispositif implantable, la matrice (4) cellulaire de réparation comprenant un volume de cellules ostéochondrogéniques prévu pour se multiplier dans l'espace de développement cellulaire (40) du dispositif implantable.

14. Implant selon la revendication 13, dans lequel la matrice (4) cellulaire de réparation comprend au moins un greffon de périoste, de préférence un greffon de périoste vascularisé, de préférence dans lequel le greffon de périoste est issu d'un périoste de tibia.

15. Implant selon l'une quelconque des revendications 13 ou 14, dans lequel l'implant est configuré pour être implanté de manière amovible dans une région intra-musculaire d'un individu et/ou est configuré pour être implanté de manière amovible dans une région sous-cutanée d'un individu.

## Patentansprüche

1. Abnehmbare implantierbare Vorrichtung, die zur Herstellung von Gelenkknorpel bestimmt ist, umfassend:
• ein erstes Trägerteil (2) aus biokompatiblem Material,
• ein zweites Trägerteil (3) aus biokompatiblem Material, das beweglich an dem ersten Trägerteil (2) gelagert ist, wobei das erste Trägerteil (2) und das zweite Trägerteil (3) zwischen einander einen Hohlraum definieren, der einen Zellentwicklungsraum (40) bildet,
wobei der Zellentwicklungsraum (40) zum Aufnehmen von osteochondrogenen Zellen vorgesehen ist, die sich in dem Zellentwicklungsraum (40) vermehren,
• externe Mobilisierungsmittel (5A, 5B), die betätigbar sind und dazu ausgestaltet sind, das zweite Trägerteil (3) derart in Bezug auf das erste Trägerteil (2) zu bewegen, das eine Scherung innerhalb des Zellentwicklungsraums (40) erzeugt wird.

2. Vorrichtung nach Anspruch 1, wobei mindestens eines von dem ersten Trägerteil (2) und dem zweiten Trägerteil eine Trägerbodenwand umfasst und zwei Trägerseitenwände (28) umfasst, die sich von der Trägerbodenwand aus erstrecken, wobei die beiden Trägerseitenwände (28) den Zellentwicklungsraum (40) begrenzen.

3. Vorrichtung nach einem der Ansprüche 1 oder 2, wobei die Vorrichtung ein geschlossenes Gehäuse umfasst, das von dem ersten Trägerteil (2) und dem zweiten Trägerteil (3) gebildet ist, wobei der Zellentwicklungsraum (40) im Inneren des Gehäuses definiert ist.

4. Vorrichtung nach einem der Ansprüche 1 bis 3, wobei das erste Trägerteil (2) eine Innenfläche (26) umfasst, die dem zweiten Trägerteil (3) zugewandt ist, wobei das zweite Trägerteil (3) dazu ausgestaltet ist, in Bezug auf das erste Trägerteil (2) entlang der Innenfläche (26) zu gleiten, wobei das zweite Trägerteil (3) vorzugsweise in Bezug auf das erste Trägerteil (2) entlang einer Richtung parallel zu einer Verlängerungsoberfläche (P) der Innenfläche (26) translatorisch beweglich gelagert ist.

5. Vorrichtung nach einem der Ansprüche 1 bis 4, wobei das zweite Trägerteil (3) in Bezug auf das erste Trägerteil (2) um eine Drehachse (R) drehbeweglich gelagert ist.

6. Vorrichtung nach Anspruch 5, wobei mindestens eines von dem ersten Trägerteil und/oder dem zweiten Trägerteil (3) eine zylinderförmige Seitenfläche (38) aufweist, die sich um die Drehachse (R) erstreckt, wobei das Trägerteil (3) vorzugsweise ferner eine Basis (39) umfasst, die im Wesentlichen senkrecht zur Seitenfläche (38) ist, wobei die äußeren Mobilisierungsmittel (5) einen Griff (54) umfassen, der drehfest mit der Basis (39) verbunden und um die Drehachse (R) drehbeweglich ist.

7. Vorrichtung nach einem der Ansprüche 1 bis 6, wobei die externen Mobilisierungsmittel mindestens ein Mobilisierungsband (5A) umfassen, das an dem zweiten Trägerteil (3) angebracht und dazu ausgestaltet ist, das zweite Trägerteil (3) zu ziehen.

8. Vorrichtung nach Anspruch 7, wobei das Mobilisierungsband (5A) an einer ersten Außenfläche (30) des zweiten Trägerteils (3) angebracht ist, wobei die Vorrichtung ferner eine zusätzliche Mobilisierungsverbindung (5B) umfasst, die an einer zweiten Außenfläche (32) des zweiten Trägerteils (3) angebracht ist, wobei die zweite Außenfläche (32) der ersten Außenfläche (30) entgegengesetzt ist.

9. Vorrichtung nach einem der Ansprüche 7 oder 8, wobei das Mobilisierungsband (5A, 5B) einen halbstarren Strang, vorzugsweise einen Strang aus Polymermaterial, umfasst.

10. Vorrichtung nach einem der Ansprüche 1 bis 9, wobei die Vorrichtung eine Schiene (6) umfasst, die dazu ausgestaltet ist, eine Verlagerung des zweiten Trägerteils (3) in Bezug auf das erste Trägerteil (2) zu führen.

11. Vorrichtung nach einem der Ansprüche 1 bis 10, wobei mindestens eines von dem ersten Trägerteil (2) und/oder dem zweiten Trägerteil (3) ein Gitter (42) umfasst, wobei das Gitter (42) eine Vielzahl von Zellwachstumshohlräumen definiert, die an einer Innenseite der Vorrichtung münden.

12. Vorrichtung nach einem der Ansprüche 1 bis 11, wobei mindestens eines von dem ersten Trägerteil (2) und/oder dem zweiten Trägerteil (3) ein Netzwerk von Versteifungselementen (7) umfasst, die an dem Trägerteil befestigt sind, wobei eine Steifigkeit gegen die Scherung an den Versteifungselementen (7) strikt größer ist als eine Steifigkeit gegen die Scherung an einer zentralen Zone des Zellentwicklungsraums (40).

13. Implantat zur Bildung von Knorpelzellen, wobei das Implantat umfasst:
• eine implantierbare Vorrichtung (1) nach einem der Ansprüche 1 bis 12,
• eine zelluläre Reparaturmatrix (4), die zwischen dem ersten Trägerteil (2) der implantierbaren Vorrichtung und dem zweiten Trägerteil (3) der implantierbaren Vorrichtung angeordnet ist, wobei die zelluläre Reparaturmatrix (4) ein Volumen von osteochondrogenen Zellen umfasst, das zur Vermehrung in dem Zellentwicklungsraum (40) der implantierbaren Vorrichtung vorgesehen ist.

14. Implantat nach Anspruch 13, wobei die zelluläre Reparaturmatrix (4) mindestens ein Periosttransplantat, vorzugsweise ein vaskularisiertes Periosttransplantat, umfasst, wobei das Periosttransplantat vorzugsweise von einem Tibia-Periost stammt.

15. Implantat nach einem der Ansprüche 13 oder 14, wobei das Implantat dazu ausgestaltet ist, entfernbar in einer intramuskulären Region eines Individuums implantiert zu werden, und/oder dazu ausgestaltet ist, entfernbar in einer subkutanen Region eines Individuums implantiert zu werden.

## Claims

1. A removable implantable device intended for the production of articular cartilage, comprising:
• a first support part (2) made of biocompatible material,
• a second support part (3) made of biocompatible material and movably mounted on the first support part (2), the first support part (2) and the second support part (3) defining therebetween a cavity that forms a cell-development space (40),
the cell-development space (40) being intended to receive osteochondrogenic cells which multiply in the cell-development space (40),
• operable external mobilisation means (5A, 5B) configured to move the second support part (3) relative to the first support part (2) so as to generate shear inside the cell-development space (40).

2. The device according to claim 1, wherein at least one of the first support part (2) and the second support part comprises a bottom support wall and comprises two side support walls (28) extending from the bottom support wall, said two side support walls (28) delimiting the cell-development space (40).

3. The device according to any one of claims 1 or 2, wherein the device comprises a closed housing, formed by the first support part (2) and the second support part (3), the cell-development space (40) being defined inside the housing.

4. The device according to any one of claims 1 to 3, wherein the first support part (2) comprises an internal face (26) facing the second support part (3), the second support part (3) being configured to slide relative to the first support part (2) along the internal face (26), preferably wherein the second support part (3) is movably mounted in translation relative to the first support part (2), in a direction parallel to an extension surface (P) of the internal face (26).

5. The device according to any one of claims 1 to 4, wherein the second support part (3) is rotatably mounted relative to the first support part (2) around an axis of rotation (R).

6. The device according to claim 5, wherein at least one of the first support part and/or the second support part (3) comprises a cylindrical side face (38) extending around the axis of rotation (R), preferably wherein said support part (3) further comprises a base (39) substantially perpendicular to the side face (38), the external mobilisation means (5) comprising a handle (54) constrained to rotate with the base (39) and rotatable around the axis of rotation (R).

7. The device according to any one of claims 1 to 6, wherein the external mobilisation means comprise at least one mobilisation lace (5A) mounted on the second support part (3), configured to tow the second support part (3).

8. The device according to claim 7, wherein the mobilisation lace (5A) is mounted on a first external face (30) of the second support part (3), the device further comprising an additional mobilisation link (5B) mounted on a second external face (32) of the second support part (3), the second external face (32) being opposite the first external face (30).

9. The device according to any one of claims 7 or 8, wherein the mobilisation lace (5A, 5B) comprises a semi-rigid strand, preferably a strand made of polymer material.

10. The device according to any one of claims 1 to 9, the device comprising a slide (6) configured to guide a movement of the second support part (3) relative to the first support part (2).

11. The device according to any one of claims 1 to 10, wherein at least one of the first support part (2) and/or the second support part (3) comprises a grid (42), the grid (42) defining a plurality of cell-growth cavities opening on an inner side of the device.

12. The device according to any one of claims 1 to 11, wherein at least one of the first support part (2) and/or the second support part (3) comprises a network of rigidifying elements (7) fixed to said support part, a rigidity against shear at the rigidifying elements (7) being strictly greater than a rigidity against shear in a central region of the cell-development space (40).

13. An implant for the formation of cartilage cells, the implant comprising:
• an implantable device (1) according to any one of claims 1 to 12,
• a cellular repair matrix (4), interposed between the first support part (2) of the implantable device and the second support part (3) of the implantable device, the cellular repair matrix (4) comprising a volume of osteochondrogenic cells intended to multiply in the cell-development space (40) of the implantable device.

14. The implant according to claim 13, wherein the cellular repair matrix (4) comprises at least one periosteal graft, preferably a vascularised periosteal graft, preferably wherein the periosteal graft comes from a periosteum of the tibia.

15. The implant according to any one of claims 13 or 14, wherein the implant is configured to be removably implanted in an intramuscular region of an individual and/or is configured to be removably implanted in a subcutaneous region of an individual.
